# EUROPEAN PATENT APPLICATION

(11) **EP 4 796 928 A1**
(43) Date of publication of application: **26.08.2026**
(21) Application number: 25382155.7
(22) Date of filing: 20.02.2025
(51) Int. Cl.: G01N 33/68

(54) **BACE-1 IN BRAIN-DERIVED EXTRACELLULAR VESICLES AS A BIOMARKER FOR ALZHEIMER'S DISEASE**

(71) Applicant: Universitat Autònoma de Barcelona, 08193 Bellaterra (Cerdanyola del Vallès) (ES)
(72) Inventor: PIVIDORI GURGO, Maria Isabel, 08393 CALDES D'ESTRAC (ES); ROSSI, Rosanna, 08012 BARCELONA (ES); MARTÍ RIPOLL, Mercè, 08340 VILASSAR DE MAR (ES)
(74) Representative: ZBM Patents - Zea, Barlocci & Markvardsen

(57) **Abstract**

The present disclosure relates to an *in vitro* method for diagnosing Alzheimer's disease (AD) in a subject, the method comprising a step of determining the level of β-secretase 1 protein (BACE-1) comprised in a population of brain-derived extracellular vesicles (BDEVs) in an isolated sample obtained from the subject, particularly wherein the population of BDEVs carry neuroligin-3 (NLGN3), wherein when the level of BACE-1 is equal to or higher than a reference this is indicative of a positive diagnosis of Alzheimer's disease. Also provided herein are methods of determining the classification of a subject according to the A/T/N system, methods for recommending a medical regimen, methods for determining therapeutic efficacy of a medical regiment, as well as methods of treatment for AD and AD-associated pathologies in a subject. Finally, the present disclosure provides kits and devices comprising: a) means for capturing a population of brain-derived extracellular vesicles; b) means for detecting the presence and/or for determining the level of BACE-1 protein; and optionally, c) a solid support.

## Description

### Technical Field

The present disclosure relates to the field of medicine, in particular to the field of protein biomarkers and their use in the diagnosis and/or prognosis of neurodegenerative diseases, particularly Alzheimer's Disease (AD). Provided herein are protein biomarkers present in extracellular vesicles, particularly brain-derived extracellular vesicles, and diagnostic and prognostic methods for Alzheimer's Disease (AD) and AD-associated pathological changes. The disclosure also relates to kits and devices for determining levels of protein biomarkers carried by brain-derived extracellular vesicles, as well as their use. Such compositions and methods are useful for preventing and/or treating AD and AD-associated pathologies.

### Background Art

According to the World Health Organization, dementia is currently the seventh leading cause of death and one of the major causes of disability and dependency among older people globally. In 2020, dementia affected over 55 million people worldwide, with the global burden of dementia expected to increase considerably in the next 25 years, due to the rise in life expectancy. By 2030, dementia is estimated to affect 78 million people, with this number expected to rise to 139 million people by 2050. Alzheimer's disease (AD) is the most common cause of dementia, accounting for 60 to 80% of dementias in humans over 65 years of age.

No effective treatments are yet available to reverse the AD pathological process, a challenge that is compounded by the fact that, by the time AD is diagnosed, neurodegeneration is widespread, rendering most treatments ineffective. Adding to this, the development of effective therapies has also been hampered by the lack of reliable biomarkers and methods to assess the efficacy of pharmacological or other therapeutic interventions.

AD shares neuropathological and symptomatic features with other diseases. As a consequence, a clear diagnosis is currently only confirmed following post-mortem analysis of brain samples from the patient. Thus, another shortcoming is evident in the fact that postmortem analysis demonstrates that 20%-30% of AD patients are misdiagnosed, instead suffering from other types of dementia, leading to such patients receiving sub-optimal medical care and being administered inadequate or ineffective therapies.

In the case of AD, the onset of molecular mechanisms that drive neuronal dysfunction and subsequent neuronal death is currently thought to occur decades before the onset of detectable symptoms. However, none of the current diagnostic tests can diagnose AD early in the pathological process and there are currently no methods to identify patients on the AD continuum at a time point before extensive neuronal death has occurred, which represents the time window when disease modifying therapies would be most effective.

Current clinical diagnostic methods of AD combine clinical testing, brain-imaging and the quantification of CSF biomarkers, thus requiring procedures that are both expensive and invasive. Moreover, there are no reliable, minimally invasive assays to evaluate the efficacy of a therapeutic regimen for the treatment of AD or AD-associated pathologies. Such an assay would greatly accelerate the development of suitable therapies.

Thus, there remains an urgent need for minimally-invasive assays that are reliable, specific and sensitive for diagnosing AD and AD-associated conditions with high specificity and good sensitivity and/or for identifying subjects at high risk of developing full-blown clinical AD.

### Summary of Invention

The present disclosure addresses the above needs by providing accurate, sensitive, specific and non-invasive methods for detecting biomarkers that are diagnostic and prognostic for AD. The present invention further provides novel methods, assays, kits and devices for diagnosing, prognosing, predicting, preventing and treating AD and MCI.

The inventors have surprisingly found that the levels of brain-derived β-secretase 1 protein (BACE-1; Uniprot ID: P56817) in plasma are diagnostic for AD. In particular, the inventors have unexpectedly found that AD may be diagnosed with high specificity and high sensitivity by means of a non-invasive, cost-effective test, namely by determining the levels of BACE-1 in a population of brain-derived extracellular vesicles (BDEVs) present in plasma, particularly in BDEVs that carry neuroligin-3 (NLGN3; Uniprot ID: Q9NZ94), a membrane protein specifically expressed in brain cells, whose expression is enriched in neuronal cells.

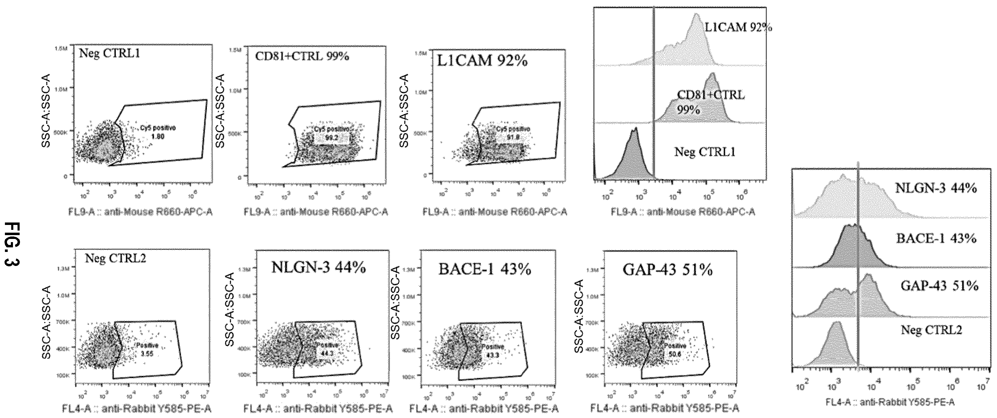

Interestingly, the inventors have found that the levels of NLGN3 in exosomes secreted by cultured neuroblastoma cells are higher than the levels of NLGN3 on the membrane of parental cells (FIGs. 3, 4 and 5), while the opposite was the case for L1CAM (FIGs. 3, 4 and 5), a membrane protein commonly employed to isolate brain-derived EV populations in prior art disclosures, suggesting that NLGN3 is enriched on the membrane of certain populations of BDEVs during exosome biogenesis, making this protein of particular interest as an EV surface marker that may be employed to immobilise, capture, concentrate, purify and/or isolate BDEV populations of interest from isolated biological fluids. Completing the present disclosure, the inventors have further surprisingly found that BACE-1 is enriched on the membrane of a population of BDEVs, while this was not true for other membrane proteins previously linked with AD, such as Rab27b or GAP-43, despite the fact that both of these have been disclosed to be overexpressed in the AD brain and the fact that the latter is directly implicated in EV biogenesis and secretion, as disclosed in prior art documents.

The inventors have thus developed an *in vitro* method comprising determining the level of BACE-1 in a population of BDEVs that may be employed for the diagnosis of AD, and/or identifying a subject with Alzheimer's disease-associated neuropathological changes, and/or determining the classification of a subject under the A/T/N system, particularly determining the amyloid biomarker classification status of a subject under the A/T/N system, as well as for predicting and monitoring the rate of cognitive decline, in particular memory decline, more in particular AD-associated memory decline in a subject, and/or monitoring therapeutic efficacy of medical interventions. Further disclosed herein is a particular combination of NLGN3, as a surface marker of a sub-population of BDEVs, and BACE-1, as an AD biomarker, useful for the diagnosis of AD, classification of subjects and monitoring therapeutic efficacy of medical interventions.

Thus, the present invention relates to minimally invasive, biomarker-based diagnostics for AD, methods to identify patients with AD-related cognitive decline or neuropathological changes, methods to classify subjects, and methods to monitor memory decline in a subject, as well as to methods, kits and devices for determining the levels of BACE-1 in a population of BDEVs present in an isolated biological sample obtained from a subject.

The methods herein disclosed offer higher specificity, sensitivity and accuracy compared to prior art methods, for example, to methods comprising determining overall BACE-1 levels in plasma or to methods comprising determining BACE-1 activity. The present disclosure further provides methods that are faster and require fewer resources to implement compared to prior art methods, thus allowing improved accessibility and cost-efficiency. Importantly, the methods of the present disclosure allow the diagnosis of Alzheimer's disease at an earlier stage in the disease process, when disease-modifying therapeutic interventions are likely to be most effective, compared to prior art methods. By detecting BACE-1 protein levels, rather than BACE-1 activity, more robust/reliable methods are provided herein, e.g. higher reproducibility is achieved, compared to prior art methods. The skilled person is aware that determining BACE-1 activity requires complex protocols and that *in vitro* enzyme activity may not represent a robust diagnostic readout, as enzyme activity is affected by myriad factors, thus adversely affecting reproducibility and reliability of the diagnostic test. For example, BACE-1 activity depends on post-translational modifications, e.g., glycosylation, phosphorylation, palmitoylation and acetylation, which may be lost or modified in plasma. It is further widely acknowledged that enzyme activity levels are highly sensitive to experimental conditions. Thus, assessing enzyme activity requires comparison with adequate controls that are run in parallel each time, further increasing the time, cost and complexity required to obtain the relevant measurements.

Furthermore, the methods of the present disclosure are amenable to miniaturisation, which lowers sample volume requirements and enables higher throughput, which further makes possible the use of the methods disclosed herein for use in clinical trials, as well as for prospective screening of large populations. Miniaturisation further allows the implementation of the methods disclosed herein on Point-of-Care (PoC) devices, thus allowing for the methods to be carried out outside of a hospital environment, such as a general care medical environment, or in a care home.

In a first aspect, the present disclosure provides an *in vitro* method for diagnosing Alzheimer's disease in a subject, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein when the level of BACE-1 is higher than a reference this is indicative of a positive diagnosis of Alzheimer's disease.

The *in vitro* method for diagnosing Alzheimer's disease disclosed in the first aspect may comprise the following steps:
a) determining the level of β-secretase 1 protein (BACE-1) comprised in a population of brain-derived extracellular vesicles (BDEVs) in the isolated sample obtained from the subject, particularly wherein the population of brain-derived extracellular vesicles (BDEVs) carry neuroligin-3 (NLGN3);
b) comparing the level of BACE-1 to a reference; and
c) diagnosing the subject with Alzheimer's disease.

A second aspect of the present disclosure relates to an *in vitro* method for determining whether a subject with dementia suffers from Alzheimer's disease-associated dementia, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein when the level of BACE-1 is higher than a reference this is indicative that the subject suffers from Alzheimer's disease-associated dementia.

A third aspect of the present disclosure relates to an *in vitro* method for deciding or recommending whether a subject should undergo further testing that may support a diagnosis of AD, in particular testing for amyloid, Tau and neuronal degeneration biomarkers required for classification of the subject according to the A/T/N system, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein when the level of BACE-1 is higher than a reference this is indicative of a positive decision or positive recommendation that the subject undergoes further testing, in particular testing for amyloid, Tau and neuronal degeneration biomarkers required for classification of the subject according to the A/T/N system.

A fourth aspect of the present disclosure relates to an *in vitro* method for classification of a subject as A+ (amyloid positive) or A- (amyloid negative) according to the A/T/N system or for determining the amyloid biomarker classification of a subject according to the A/T/N system, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein when the level of BACE-1 is higher than a reference this is indicative of a classification as A+ of the subject according to the A/T/N system, whereas when the level of BACE-1 is equal to or lower than the reference this is indicative of a classification as A- of the subject according to the A/T/N system. The skilled person is aware that all A/T/N biomarker combinations, also known as A/T/N biomarker categories or as A/T/N classification groups, with A+ reflect a neuropathological change related to the Alzheimer's disease continuum.

A fifth aspect of the present disclosure relates to an *in vitro* method for determining whether a subject is positive for the amyloid biomarker category and for at least one other biomarker category according to the A/T/N classification system, the method comprising a step of determining the level of β-secretase 1 protein (BACE-1) comprised in a population of brain-derived extracellular vesicles (BDEVs), in an isolated sample obtained from the subject, particularly wherein the population of brain-derived extracellular vesicles (BDEVs) carry neuroligin-3 (NLGN3), wherein when the level of BACE-1 is higher than a reference this is indicative that the subject is positive for the amyloid biomarker category and for at least one other biomarker category according to the A/T/N classification system. Thus, the fifth aspect provides an *in vitro* method for determining whether a subject falls within one of the following classification groups under the A/T/N system: A+T+N+, A+T+N-, A+T-N+, or A+T-N-.

A sixth aspect of the present disclosure relates to an *in vitro* method for monitoring a change of classification of a subject from A- to A+ under the A/T/N classification system, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein when the level of BACE-1 is higher than a reference, but the level of BACE-1 determined for the same subject at an earlier time point was equal to or lower than the reference, this is indicative that the classification of the subject under the A/T/N system has changed from A- to A+ since the earlier time point.

A seventh aspect of the present disclosure relates to an *in vitro* method for predicting a rate of memory decline in a subject without dementia, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein when the level of BACE-1 is higher than a reference this is predictive of a rate of memory decline that is higher than the rate of memory decline observed in an age-matched control subject.

An eighth aspect of the present disclosure relates to an *in vitro* method for monitoring a rate of memory decline in a subject without dementia, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein a change in the level of BACE-1 is indicative of the rate of memory decline in the subject, wherein the change in the level of BACE-1 is calculated as the difference between the level of BACE-1 determined in the isolated sample obtained from the subject and the level of BACE-1 determined in an isolated sample from the same subject at an earlier time point.

A ninth aspect of the present disclosure relates to an *in vitro* method for predicting a pathological rate of memory decline in a subject without dementia, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein: (i) when the level of BACE-1 is higher than a reference this is indicative of a pathological rate of memory decline in the subject, or, alternatively, (ii) when an increase in the level of BACE-1 in the isolated sample obtained from the subject, compared to the level of BACE-1 determined in an isolated sample obtained from the same subject at an earlier time point, is higher than an increase in the level of BACE-1 in an isolated sample obtained from an age-matched control subject, compared to the level of BACE-1 determined in an isolated sample obtained from the same age-matched control subject at the earlier time point, this is indicative of a pathological rate of memory decline in the subject.

A tenth aspect of the present disclosure relates to an *in vitro* method for identifying a subject with Alzheimer's disease-associated neuropathological changes, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein when the level of BACE-1 is higher than a reference this is indicative of Alzheimer's disease-associated neuropathological changes in the subject.

An eleventh aspect of the present disclosure relates to an *in vitro* method of stratifying a subject with Alzheimer's disease according to disease severity, the method comprising a step of determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein the level of BACE-1 is indicative of Alzheimer's disease severity.

In an twelfth aspect, the present disclosure provides an *in vitro* method for determining efficacy of a medical regimen in a subject with Alzheimer's disease or in a subject at risk of developing Alzheimer's disease or in a subject with Alzheimer's disease-associated neuropathological changes or in a subject with Alzheimer's disease-associated dementia or in a subject with a pathological rate of memory decline, the method comprising determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), wherein, when the level of BACE-1 is lower than the level of BACE-1 comprised in brain-derived extracellular vesicles (BDEVs) determined in an isolated sample obtained from the same subject before the start of the medical regimen or at an earlier phase of the medical regimen, this is indicative of efficacy.

Also provided herein, in a thirteenth aspect, is a method of delaying Alzheimer's disease onset in a subject, the method comprising: (i) determining the level of β-secretase 1 protein (BACE-1) comprised in a population of brain-derived extracellular vesicles (BDEVs) in an isolated sample obtained from the subject, particularly wherein the population of brain-derived extracellular vesicles (BDEVs) carry neuroligin-3 (NLGN3), and (ii) administering a medical regimen for the treatment of Alzheimer's disease to the subject, if the level of BACE-1 is higher than a reference.

In a fourteenth aspect, the present disclosure relates to a method of treating Alzheimer's disease or Alzheimer's disease-associated dementia in a subject in need of thereof, the method comprising: diagnosing Alzheimer's disease by an *in vitro* method as defined in the first aspect above or in the twenty-seventh or twenty-eighth aspects below, or determining that the subject suffers from Alzheimer's disease-associated dementia by an *in vitro* method as defined in the second aspect, or identifying that the subject suffers from Alzheimer's disease-associated neuropathological changes as defined in the tenth aspect, and administering a medical regimen for the treatment of Alzheimer's disease or for the treatment of Alzheimer's disease-associated dementia or for the treatment of Alzheimer's disease-associated pathological changes to the subject, if the subject is diagnosed as having Alzheimer's disease or the subject is determined to suffer from Alzheimer's disease-associated dementia or the subject is identified as suffering from Alzheimer's disease-associated neuropathological changes.

Furthermore, a fifteenth aspect of the present disclosure relates to a method of deciding or recommending whether to initiate a medical regimen in a subject suspected of suffering from Alzheimer's disease or of being at risk of developing Alzheimer's disease, the method comprising the steps of: (i) diagnosing Alzheimer's disease in the subject by using an *in vitro* method as defined in the first aspect above or in the twenty-seventh or twenty-eighth aspects below, or determining that the subject suffers from Alzheimer's disease-associated dementia by an *in vitro* method as defined in the second aspect; and (ii) deciding or recommending to initiate the medical regimen if the subject is diagnosed as suffering from Alzheimer's disease or the subject is determined to suffer from Alzheimer's disease-associated dementia.

A further, sixteenth, aspect of the present disclosure relates to an *in vitro* method of deciding or recommending whether to initiate a medical regimen in a subject, the method comprising the steps of: (i) classifying the subject as A+ (amyloid positive) according to the A/T/N system by using an *in vitro* method as defined in the fourth aspect; or, alternatively, determining that the subject is positive for the amyloid biomarker category and for at least one other biomarker category according to the A/T/N classification system by using an *in vitro* method as defined in the fifth aspect; or, alternatively, determining whether a subject with dementia suffers from Alzheimer's disease-associated dementia by using an *in vitro* method as defined in the second aspect; or, alternatively, predicting a pathological rate of memory decline in the subject by using an *in vitro* method as defined in the ninth aspect; or, alternatively, identifying the subject as a subject with Alzheimer's disease-associated neuropathological changes by using an *in vitro* method as defined in the tenth aspect; and (ii) deciding or recommending to initiate the medical regimen if the subject is, respectively, classified as A+ (amyloid positive) according to the A/T/N system, or is determined to be positive for the amyloid biomarker category and for at least one other biomarker category according to the A/T/N classification system, or is determined to suffer from Alzheimer's disease-associated dementia, or is predicted to undergo a pathological rate of memory decline, or is identified as a subject with Alzheimer's disease-associated neuropathological changes.

In a seventeenth aspect, the disclosure provides an *in vitro* method of determining the level of brain-derived β-secretase 1 protein (BACE-1) comprised in an isolated peripheral biological fluid sample obtained from a subject, the method comprising the steps of: (i) capturing a population of brain-derived extracellular vesicles (BDEVs) comprised within the isolated peripheral biological fluid sample, particularly wherein the population of brain-derived extracellular vesicles (BDEVs) carry neuroligin-3 (NLGN3); and (ii) determining the level of BACE-1 comprised in the population of brain-derived extracellular vesicles (BDEVs) captured in step (i).

An eighteenth aspect of the disclosure provides a kit comprising: a) means for capturing a population of brain-derived extracellular vesicles (BDEVs), particularly means for capturing a population of brain-derived extracellular vesicles (BDEVs) that carry neuroligin-3 (NLGN3); and b) means for detecting the presence and/or for determining the level of BACE-1 protein.

A further, nineteenth, aspect of the present disclosure relates to a device comprising: a) optionally, a solid support; b) means for capturing a population of brain-derived extracellular vesicles (BDEVs), particularly means for capturing a population of brain-derived extracellular vesicles (BDEVs) that carry neuroligin-3 (NLGN3); and c) means for detecting the presence and/or for determining the level of BACE-1 protein. The device of the nineteenth aspect may be a point-of-care (PoC) device, particularly a PoC device that comprises a microfluidic chip.

In a twentieth aspect, the present disclosure relates to use of a kit and/or device according to the eighteenth and/or nineteenth aspects in a method as defined in any one of the first to the seventeenth aspects.

A twenty-first aspect of the present disclosure relates to a combined use of BACE-1 protein and a marker for a population of brain-derived extracellular vesicles (BDEVs) in an *in vitro* method as defined in any one of the first to the seventeenth aspects, wherein the marker for the population of BDEVs is a neuronal-enriched membrane protein, particularly wherein the neuronal-enriched membrane protein is neuroligin-3 (NLGN3).

In a twenty-second aspect, the present disclosure relates to use of BACE-1 protein comprised in a population of brain-derived extracellular vesicles (BDEVs) as a biomarker for diagnosing Alzheimer's disease, or for determining whether a subject with dementia suffers from Alzheimer's disease-associated dementia, or for identifying a subject with Alzheimer's disease-associated pathological changes, or for deciding or recommending whether a subject should undergo testing for amyloid, Tau and neuronal degeneration biomarkers required for classification of the subject according to the A/T/N system, or for classification of a subject as A+ (amyloid positive) or A- (amyloid negative) according to the A/T/N system, or for determining whether a subject is positive for the amyloid biomarker category and for at least one other biomarker category according to the A/T/N classification system, or for monitoring a change of classification of a subject from A- to A+ under the A/T/N classification system, or for predicting or monitoring a rate of memory decline in a subject without dementia, or for predicting a pathological rate of memory decline in a subject without dementia, or for stratifying a subject with Alzheimer's disease according to disease severity, or for determining efficacy of a medical regimen in a subject with Alzheimer's disease or in a subject at risk of developing Alzheimer's disease or in a subject with Alzheimer's disease-associated neuropathological changes or in a subject with Alzheimer's disease-associated dementia or in a subject with a pathological rate of memory decline.

A related, twenty-third aspect, of the present disclosure relates to use of BACE-1 comprised in a population of brain-derived extracellular vesicles (BDEVs) in an *in vitro* method of deciding or recommending whether to initiate a medical regimen in a subject suspected of suffering from Alzheimer's disease or in a subject with Alzheimer's disease-associated neuropathological changes or in a subject with Alzheimer's disease-associated dementia or in a subject with a pathological rate of memory decline, particularly wherein the population of brain-derived extracellular vesicles (BDEVs) carry neuroligin-3 (NLGN3).

Similarly, a twenty-fourth aspect discloses a use of BACE-1 comprised in a population of brain-derived extracellular vesicles (BDEVs) in an *in vitro* method for determining the efficacy of a medical regimen in a patient already diagnosed with Alzheimer's disease, particularly wherein the population of brain-derived extracellular vesicles (BDEVs) carry neuroligin-3 (NLGN3).

Furthermore, a twenty-fifth aspect relates to a use of BACE-1 comprised in a population of brain-derived extracellular vesicles (BDEVs) in a method of stratifying a subject according to risk of developing Alzheimer's disease within the 3-5 subsequent years, particularly wherein the population of brain-derived extracellular vesicles (BDEVs) carry neuroligin-3 (NLGN3).

A yet further, twenty-sixth aspect, relates to a use of BACE-1 comprised in a population of brain-derived extracellular vesicles (BDEVs) in an *in vitro* method of determining a subject's probability of developing Alzheimer's disease within the 3-5 subsequent years, particularly wherein the population of brain-derived extracellular vesicles (BDEVs) carry neuroligin-3 (NLGN3).

In a twenty-seventh aspect, the present disclosure provides an *in vitro* method for diagnosing Alzheimer's disease in a subject, the method comprising a step of determining the level of β-secretase 1 protein (BACE-1) comprised in an isolated peripheral biological fluid sample obtained from the subject, wherein the level of BACE-1 is the level of BACE-1 derived from a brain cell, particularly wherein the brain cell expresses neuroligin-3 (NLGN3), wherein when the level of BACE-1 is equal to or higher than a reference this is indicative of a positive diagnosis of Alzheimer's disease.

In a twenty-eighth aspect, the present disclosure relates to an *in vitro* method for diagnosing Alzheimer's disease in a subject, the method comprising a step of determining the level of β-secretase 1 protein (BACE-1) comprised in a population of extracellular vesicles (EVs) that carry neuroligin-3 (NLGN3) and that are comprised in an isolated sample obtained from the subject, wherein when the level of BACE-1 is equal to or higher than a reference this is indicative of a positive diagnosis of Alzheimer's disease.

### Description of Drawings

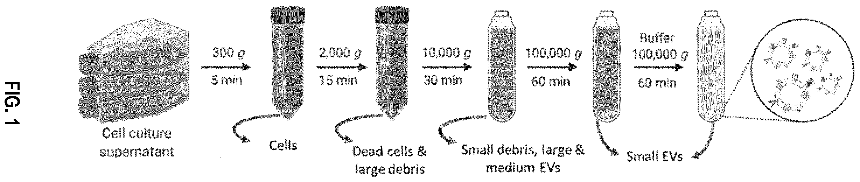

FIG. 1 shows a graphical summary of the protocol employed for the isolation and purification of extracellular vesicles (EVs) from cell culture supernatant of SH-SY5Y cells, using differential ultracentrifugation steps. The arrows indicate sequential centrifugation steps, which were carried out for the indicated durations at centrifugal speeds of 300, 2,000, 10,000 and 100,000 x g, respectively, to separate small EVs (which were obtained after the last centrifugation step), from live cells, dead cells and large debris, and small debris, large EVs and medium-sized EVs, as indicated.

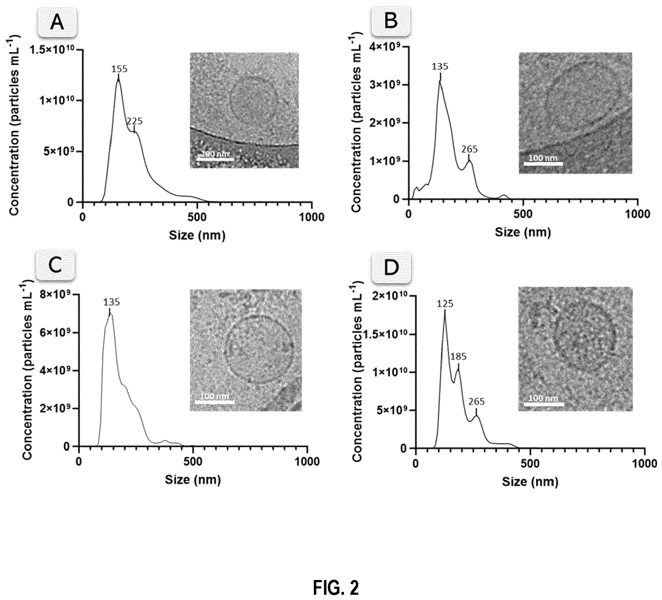

FIG. 2 shows graphs representing the results of nanoparticle tracking analysis (NTA) of exosomes, as well as insets of representative micrographs of exosomes visualised with cryo-transmission electron microscopy (Cryo-TEM). Panels A to D relate to data for purified exosomes derived from SH-SY5Y cells (panel A), plasma from healthy donors (panel B), plasma from patients with mild cognitive impairment (MCI patients; panel C) and plasma from patients with Alzheimer's disease (AD patients; panel D).
FIG. 3 shows a summary of the results obtained for the characterisation of SH-SY5Y cells by flow cytometry. Results are represented with both dot plots and histograms, indicating the percentage of cells positively labelled with the antibodies against tetraspanin CD81 (used as positive control) and against the L1CAM, NLGN3, BACE-1 and GAP-43 proteins. Negative control samples were obtained by omission of the primary antibody and using, as secondary antibody, an anti-mouse-Cy5 for Neg CTRL1 or an anti-rabbit-PE for Neg CTRL2. The perimeter of the gates used to define the populations of positively-labelled cells are marked on each graph. Gating strategies were determined with respect to the signal obtained for the relevant negative control sample in each case. For all graphs, the values plotted on the x-axis represent the intensity of the fluorescent signal detected for each event (i.e. each dot in a dot plot). For dot plots, the values plotted on the y-axis represent the side scatter (SSC) value determined for each event.

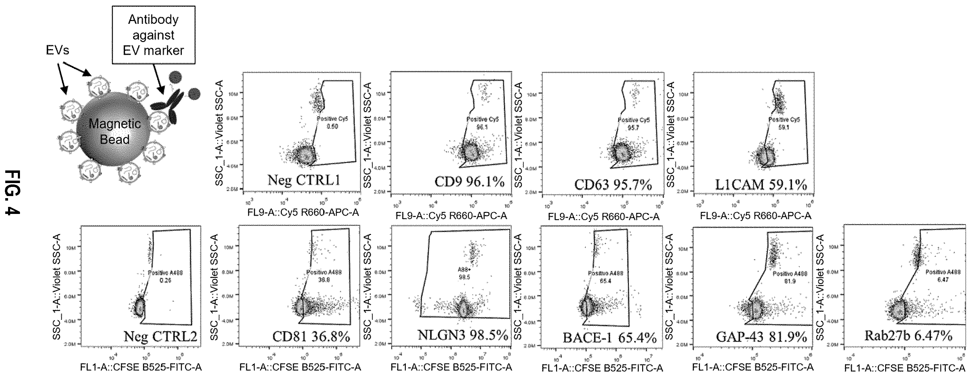

FIG. 4 shows a summary of the results obtained for the characterisation of SH-SY5Y-derived exosomes by flow cytometry. For this analysis, SH-SY5Y exosomes were directly covalently immobilised on magnetic particles, as detailed below. Shown in the upper left corner of the figure is a schematic diagram of a magnetic particle whose surface exosomes have been immobilised on. Further depicted on this diagram is a Y-shaped antibody molecule binding to a marker present on the surface of an exosome that is directly covalently immobilised on the surface of the magnetic particle. Results of characterisation of SH-SY5Y-derived exosomes by flow cytometry are represented with dot plots, wherein the intensity of the fluorescent signal and the side scatter value (SSC) determined for each event (i.e. each dot on a dot plot) are plotted on the x-axis and on the y-axis, respectively. Indicated on each plot is the percentage of positive labeling observed for each of the three tetraspanins CD9, CD63 and CD81 and for the NLGN3, BACE-1, GAP-43 and Rab-27b marker proteins. Negative control samples were obtained by omission of the primary antibody and using, as secondary antibody, an anti-mouse-Cy5 for Neg CTRL1 and an anti-rabbit-A488 for Neg CTRL2.

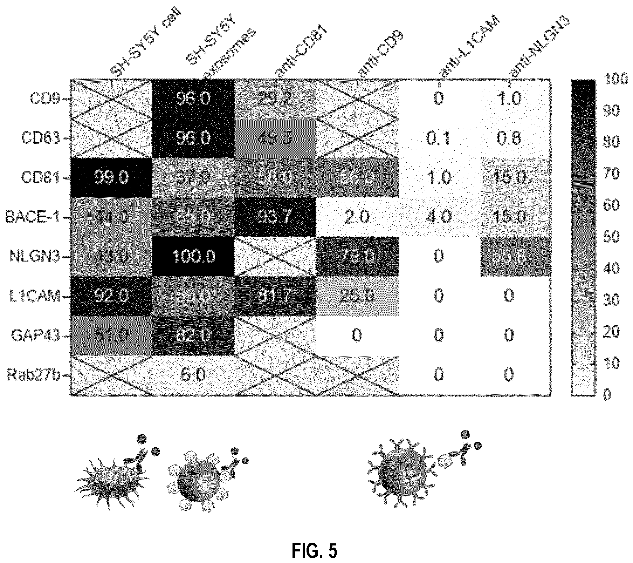

FIG. 5 shows a heatmap summary of the results obtained by bead-based flow cytometry for characterisation of SH-SY5Y cells and SH-SY5Y-derived exosomes. Column titles indicate the sample type, namely, from left to right: SH-SY5Y cells (column 1), SH-SY5Y-derived exosomes covalently immobilised on tosylated magnetic particles (column 2), and SH-SY5Y-derived exosomes captured on tosylated magnetic particles, wherein the magnetic particles were functionalised with anti-CD81, anti-CD9, anti-L1CAM, or anti-NLGN3 antibodies, respectively (columns 3-6, respectively). Row titles indicate the exosomal surface marker proteins detected in each sample with detection antibodies raised against the respective marker protein, namely, from top to bottom: CD9, CD63, CD81, BACE-1, NLGN3, L1CAM, GAP-43 and Rab27b. The numbers in each cell of the figure indicate the percentage of positive labelling observed in each sample type with detection antibodies raised against the tetraspanins CD9, CD63 and CD81, or against the different markers tested, namely BACE-1, NLGN3, L1CAM, GAP-43 and Rab27b.

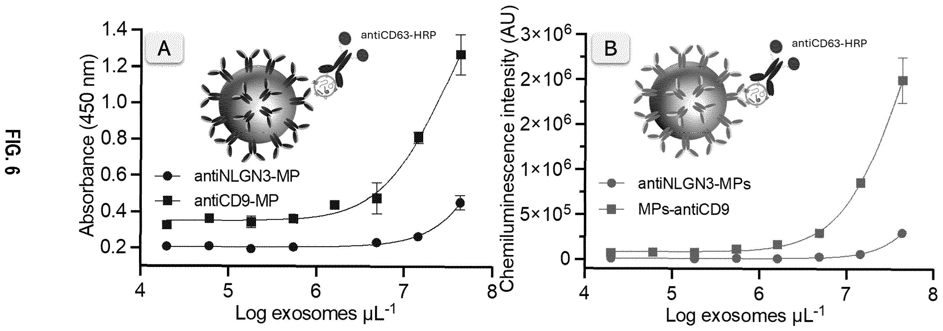

FIG. 6 shows the results of magneto-actuated immunoassays with optical (ELISA, panel A) and chemiluminescent (CHEM-ELISA, panel B) readout carried out to evaluate the capture capabilities of magnetic particles functionalised with an anti-NLGN3 antibody (antiNLGN3-MPs; circles) compared to magnetic particles functionalised with an anti-CD9 antibody (antiCD9-MPs; squares). An anti-CD63 antibody coupled with horse radish peroxidase (HRP) (antiCD63-HRP) was used for detection of the captured exosomes in both cases. SH-SY5Y-derived exosomes were used. Calibration plots were fitted using a non-linear regression (four-parameter logistic equation) (n=3).

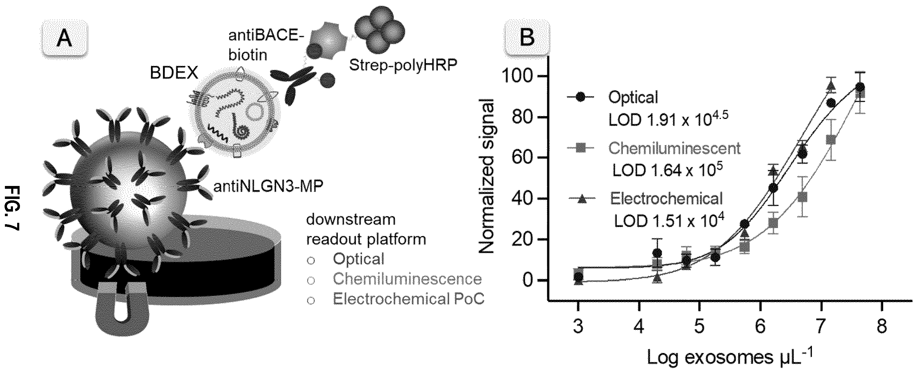

FIG. 7A shows a diagram of the principles underlying a magneto-actuated immunoassay for early diagnosis of AD in plasma. In such an assay, tosylated magnetic particles functionalized with an anti-NLGN3 antibody are used to capture brain-derived exosomes (BDEs). Detection of BACE-1 comprised in the captured BDEs uses a biotinylated anti-BACE-1 antibody in combination with streptavidin-poly-HRP (Strep-Poly-HRP) to enhance the signal. The assay may comprise a downstream readout, such as an optical, chemiluminescence or electrochemical readout.
FIG. 7B shows calibration plots for SH-SY5Y-derived exosomes analysed by magneto-actuated immunoassay as described in Fig. 7A, with different readout platforms: optical (absorbance at 450 nm; circles), chemiluminescence (squares), and electrochemical (triangles) readouts. The limits of detection (LOD) as determined for each readout platform are indicated on the graph as the number of exosomes per µL. Plots were fitted using a non-linear regression (four-parameter logistic equation) (n=3).

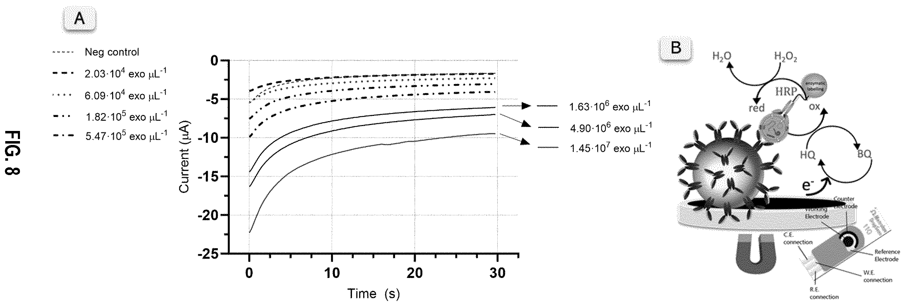

FIG. 8A shows raw chronoamperograms for exosomes derived from SH-SY5Y cells analysed by magneto-actuated assay with electrochemical readout. Each curve corresponds to a different concentration of exosomes as indicated on the graph. From top to bottom, in order, the curves correspond to the following samples: negative control, 2.03 x 10⁴ exosomes per µL, 6.09 x 10⁴ exosomes per µL, 1.82 x 10⁵ exosomes per µL, 5.47 x 10⁵exosomes per µL, 1.63 x 10⁶exosomes per µL, 4.90 x 10⁶exosomes per µL, and 1.45 x 10⁷ exosomes per µL. Curves represent the average of 3 replicates.
FIG. 8B shows a schematic diagram summarising the principles underlying the magento-actuated assay with electrochemical readout, implemented on a Point-of-Care (PoC) device, that may be used to analyse exosomes from cultured cells or to diagnose AD in an isolated biological fluid sample obtained from a patient according to particular embodiments of the present disclosure, namely through capturing brain-derived exosomes (BDEs) comprised with the isolated biological fluid sample and determining the levels of BACE-1 comprised within the captured BDEs. An assay according to this diagram was used for obtaining the data in FIG. 8A.

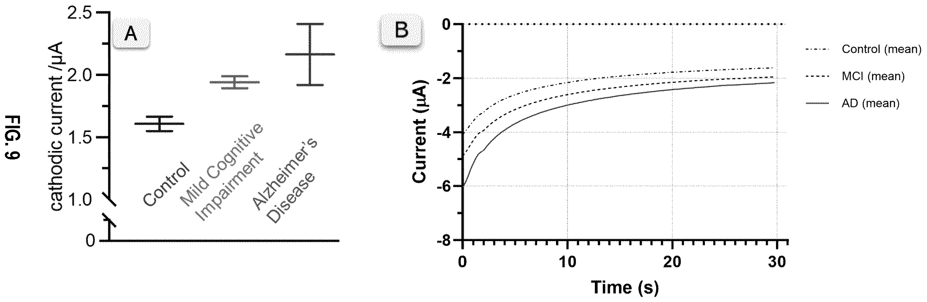

FIG. 9 shows the results of analyses carried out on exosomes comprised in plasma samples from patients with Alzheimer's disease (AD) or Mild Cognitive Impairment (MCI) compared to exosomes comprised in plasma samples from control (CTRL) subjects, where levels of BACE-1 on brain-derived exosomes in human plasma were analysed using a magneto-actuated assay implemented on a Point-of-Care (PoC) device comprising a miniaturised electrochemical platform combined with a screen-printed electrode. Results of 2 replicates are shown as bar plots (panel A; shown is the mean, with error bars indicating standard deviation) and raw chronoamperograms (panel B). In panel B, the top chronoamperogram corresponds to control subjects (dot-dash line), the middle chronoamperogram corresponds to patients with MCI (dashed line), and the bottom chronoamperogram corresponds to patients with AD (solid line).

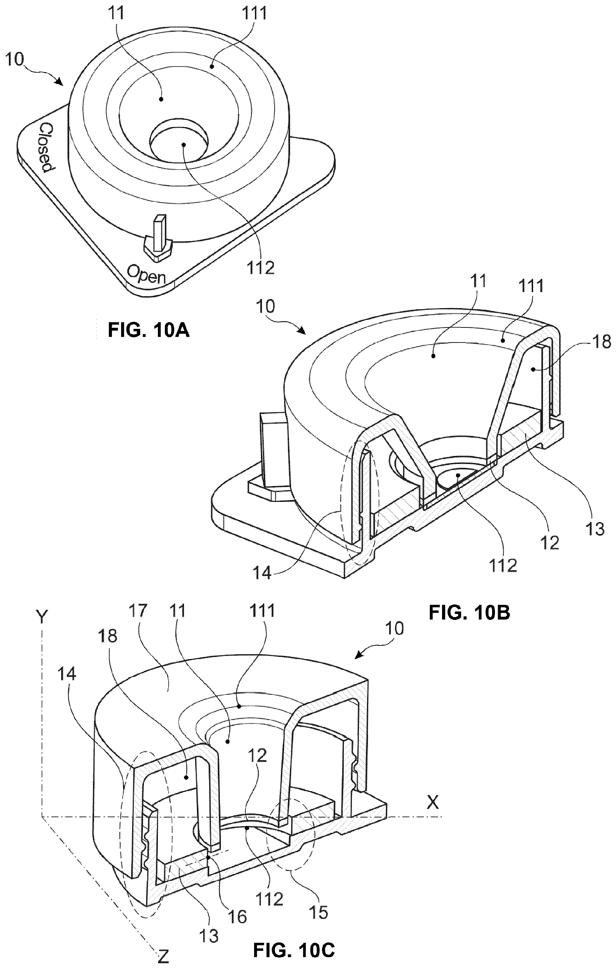

FIG. 10A shows an example representation of a device 10 or cartridge 10 according to the invention. FIG. 10A shows a chamber 11 comprising a first opening 111 and a second opening 112.
FIG. 10B shows an example representation of a section of a device 10 or cartridge 10 according to the invention in a closed position. FIG. 10B shows the device 10 comprising a chamber 11 as a cavity 11, the cavity 11 comprising a first opening 111 in a higher height and a second opening 112 in a lower height. FIG. 10B also shows the sealing element, sealing gasket or O-ring 12, the absorbing pad 13 and an opening assembly 14 in the closed position.
FIG. 10C shows an example representation of a device 10 or cartridge 10 according to the invention in an open position on a plane XZ. FIG. 10C shows the device 10 comprising a chamber 11 comprising a first opening 111, and a second opening 112. FIG. 10C also shows the sealing element 12, the absorbing pad 13, the opening assembly 14 in the open position and a liquid exit 15. The liquid exit 15 is the opening formed between the cover or casing 17 -comprising the chamber 11- and the base of the device. The device or cartridge 10 is shown on a standing or upright position where, considering the reference plane XZ, the first opening 111 is in a higher position in an axis Y perpendicular to the plane XZ than the second opening 112. In use, the upright position allows the liquids to be entered or inserted through the first opening 111; the liquids fall by gravity into the chamber and rest on the base of the chamber. In the closed position the liquid exit 15 is closed and sealed by the sealing element 12, so that the sealing element 12 prevents the liquid from exiting the chamber 11, and in the open position the liquid exit 15 is open, a fluidic path 16 is formed, letting the liquid exit the chamber 11 towards the liquid absorbing pad 13 along the fluidic path 16. Although the absorbing pad 13 is shown in FIGs. 10A, 10B, 10C having a specific width, the absorbing pad 13 may completely occupy the available space 18 shown in the FIG. 10B, for providing an improved absorbing capacity.

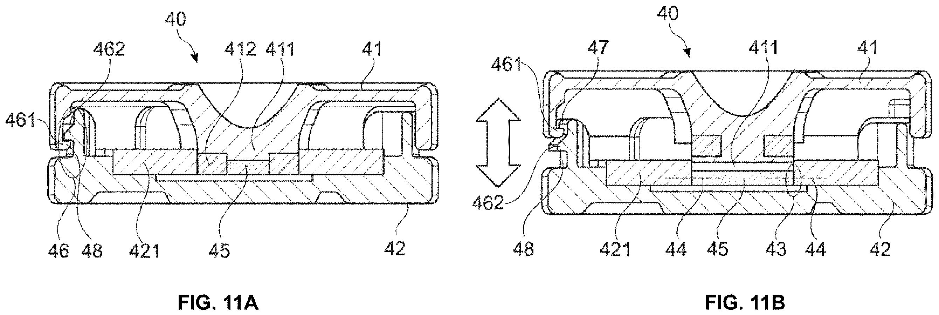

FIGs. 11A and 11B show a further example of the cartridge 40 in the closed and in the open positions respectively. The cartridge 40 comprises a first cover 41 comprising the chamber 411 and the sealing element 412. The first cover 41 fits a second cover 42. The second cover 42 comprises a liquid absorbing pad 421, wherein the liquid absorbing pad 421 faces the liquid exit 43 (seen unsealed in FIG. 11B). In this example, the liquid exit 43 is the hole or cavity 43 formed between the first cover 41 and the second cover 42 in the open position. The liquid exit 43 is covered by the sealing element 412 in the closed position and uncovered leaving a fluidic path 44 between the chamber and the absorbing pad 421 in the open position. Besides, in the closed position, the sealing element 412 cuts the fluidic path 44 between the chamber 411 and the liquid absorbing pad 421, thus preventing the liquid from exiting the chamber. As seen, in the closed position -FIG. 11A- a liquid 45 is retained in the chamber 411, whereas in the open position -FIG. 11B- the liquid 45 flows along the fluidic path 44 and is absorbed by the absorbing pad 421. Additionally, an opening assembly 46 comprises a first cooperating mechanism 461 and a second cooperating mechanism 462 wherein the first cover 41 comprises the first cooperating mechanism 461 and the second cover 42 comprises the second cooperating mechanism 462. The first cooperating mechanism 461 and the second cooperating mechanism 462 of cartridge or device 40 are moveable relative to each other such that the first cover 41 and the second cover 42 are movable relative to each other to shift between the closed position and the open position. In particular, the cooperating mechanisms in the cartridge or device 40 are first locking tab 461 and second locking tab 462. A user may raise the first cover 41 up from the second cover 42 and fix the first cover 41 in the open position positioning the first locking tab 461 on the second locking tab 462 on point 47, and a user may lower the first cover 41 down towards the second cover 42 and fix the first cover 41 in the closed position positioning the first locking tab 461 below the second locking tab 462 on point 48.

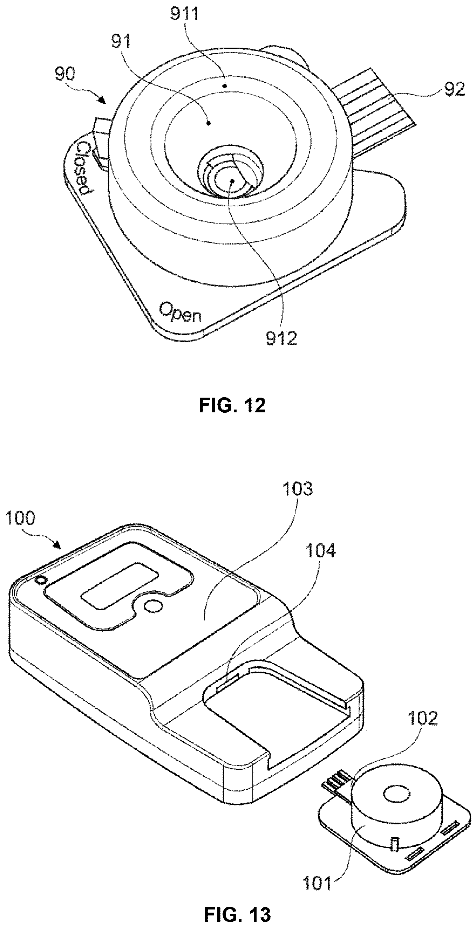

FIG. 12 shows an example device 90 or cartridge 90. FIG. 12 shows the cartridge 90 with an electrode 92 inserted through an electrode receiving slot. The inserted part of the electrode may be seen through the second opening 912 of the chamber 91. Liquids may be inserted into the chamber 91 through the first opening 911 and fall by gravity, in the upright position, to the second opening 912 where they enter into contact with the electrode 92. A magnet (not shown) located under the electrode may attract magnetic particles and retain them in an open position so that a liquid may be absorbed by an absorbing pad (not shown).
FIG. 13 shows an example assay system 100 which comprises a device 101 or cartridge 101 comprising an electrode 102, the assay system comprising a reader 103 configured to read the electrode 102, which may be inserted into an electrode slot 104 provided in the reader 103.

### Detailed description of the invention

All terms as used in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

Throughout the description and claims the word "comprise" and variations of the word, are not intended to exclude other technical features, additives, components, or steps. Furthermore, the word "comprise" encompasses the case of "consisting of".

Aspects of the present disclosure are directed to the diagnosis, stratification, classification, monitoring and treatment of subjects with neurodegenerative diseases, in particular Mild Cognitive Impairment (MCI) and/or Alzheimer's Disease (AD), more in particular early AD. As used herein, a "neurodegenerative disease" refers to a pathological condition characterised by neuronal damage causing the affected neurons to lose functionality, typically leading to the progressive loss/death of the damaged neurons. Clinical manifestations of neurodegenerative diseases vary depending on the type and location of the affected neurons, as well as the extent of neurodegeneration.

As used herein, the term "treatment" refers to a medical, pharmacological or lifestyle intervention aimed at maintaining or restoring health, which may include curative interventions as well as interventions that relieve or improve symptoms or pain and/or discomfort that the subject is experiencing as a consequence of the pathology being treated. Thus, in this sense, treatment includes prophylactic or preventative treatment, aimed at maintaining health by preventing ill effects that would otherwise arise. Furthermore, in the context of the present disclosure, treatment also includes interventions that delay the onset or progression of the pathology.

In AD dementia, memory loss, particularly loss of short-term memory, is typically one of the first symptomatic manifestations of disease. Decline in non-memory aspects of cognition, such as language dysfunction (e.g. difficulties recalling a correct word), visuospatial dysfunction (e.g. trouble understanding visual images or spatial relationships), and impaired reasoning or judgment, may also become apparent in the early stages of AD. Additional manifestations of AD are behaviour disorders such as, wandering, agitation, yelling, and persecutory ideation. Treatment of AD includes safety and support measures and may also include pharmacological treatment, for example with acetylcholinesterase inhibitors, such as Donepezil, and/or with N-Methyl-D-aspartate (NMDA) receptor agonists, such as Memantine.

Prior to clinical symptomatology, the early stages of AD manifest as Mild Cognitive Impairment (MCI), representing a transitional phase from cognitive decline associated with normal/physiological aging to those typically found in dementia. However, not all subjects with MCI progress to AD. MCI refers to a condition which is largely equivalent to that designated as Mild Neurocognitive Disorder by the Fifth Edition of the Diagnostic and Statistical Manual of Mental Disorders, DSM-5, as well as in the eleventh revision of the International Classification of Diseases, ICD-11.

From a pathophysiological perspective, AD is a neurocognitive neurodegenerative disorder, characterised by the accumulation of extracellular β-amyloid deposits/plaques and intracellular neurofibrillary Tau tangles in the cerebral cortex and subcortical grey matter. Most cases of AD are sporadic AD, where there is no discernible family link, and the disease is understood to arise from a complex combination of genetic, environmental and lifestyle factors. Vascular risk factors, e.g. smoking or other chemical toxic agents, can also increase the risk of AD. Mutations in genes encoding for the amyloid precursor protein *(APP),* as well as presenilin-1 and presenilin-2 (*PSEN1* and *PSEN2,* respectively), may lead to less frequent autosomal dominant forms of AD, typically with presenile onset (familial AD). Other genetic determinants include apolipoprotein E epsilon (*APOE* ε) alleles, which influence β-amyloid deposition, and *TREM2* mutations, which affect neuroinflammation.

At molecular level, AD progression is linked to the aforementioned accumulation of cytotoxic amyloid beta (Aβ) peptides and hyperphosphorylated Tau protein, combined with other pathological features such as synaptic loss, defective energy metabolism, and imbalances in protein and metal homeostasis. Cytotoxic Aβ peptides, consisting of 37-43 amino acids, with the Aβ42 peptide regarded as most deleterious, are produced via enzymatic processing of the amyloid precursor protein (APP) by β-secretase 1 (BACE-1) and γ-secretase, with BACE-1 representing the rate-limiting enzyme in the amyloidogenic pathway of APP processing. In human, BACE-1 is the protein corresponding to Uniprot ID: P56817. References to Uniprot ID records provided in the present disclosure refer to release 2025_01 of the UniProtKB database, published on 05 February 2025.

The term "reference" or "reference value" or "cut off" or "threshold", as used herein, relates to a predetermined criterion used as a reference for evaluating the values or data obtained from the isolated sample obtained from a subject to be tested using the methods of the present disclosure. The reference can be an absolute value, a relative value, a value that has an upper or a lower limit, a range of values, an average value, a median value, a mean value, or a value as compared to a particular control or baseline value. The reference can be based on a large number of samples, such as from a population of subjects of a chronological age-matched group or based on a pool of samples including or excluding the sample to be tested. The skilled person in the art, making use of the general knowledge, can choose the subject or group of subjects more adequate for obtaining the reference for each of the methods of the present invention. Methods for obtaining the reference from the group of subjects selected are well-known in the state of the art, for example, as disclosed in Burtis et al., 2008, Chapter 14, section "Statistical Treatment of Reference Values". In a particular case, the "reference" is a cut-off value or threshold defined, for example, by means of a conventional ROC analysis (Receiver Operating Characteristic analysis). The level of BACE-1 determined in the isolated sample obtained from the subject being tested may be lower or higher that the reference, or, where the reference is a range of values, it may fall within the range or outside the range. The skilled person in the art, making use of the general knowledge, can choose the adequate criteria for comparing the level of BACE-1 determined in the isolated sample from the subject being tested to the reference.

In the context of the present disclosure, a reference may comprise a value or a range of values for the level of BACE-1 determined in a reference sample, wherein the reference sample is obtained from a subject that is either cognitively normal (also referred to as a "control subject") or is deemed to have mild cognitive impairment, but that does not suffer from a neurodegenerative disease, including a dementia. Thus, the level of BACE-1 in the "reference sample" may be considered representative for the level of BACE-1 in an isolated sample from a non-diseased subject, so that the reference sample can serve as a comparator against which level of BACE-1 determined in the isolated sample according to the present disclosure can be compared. In this case, the subject whose isolated sample is being tested may be identified as suffering from AD, or AD-associated pathological changes, memory decline, cognitive impairment, mild cognitive impairment, or dementia, or, alternatively, may be determined as being at least amyloid-positive under the A/T/N system, or, alternatively, may be recommended to undergo further testing for classification according to the A/T/N system, or, alternatively, may be recommended to start a given medical regimen if an increase is observed in the level of BACE-1 determined according to the present disclosure in the isolated sample obtained from the subject, compared to the level of BACE-1 in the reference sample.

In the sense of the present disclosure, an increase in the level of BACE-1 compared to the reference may be, typically, of at least 10%, particularly at least 15%, more particularly at least 20%, even more particularly at least 25%, most particularly at least 30%.

In the context of the present disclosure, determining the level of BACE-1 may comprise evaluating if BACE-1 is present in the sample (detection) and/or evaluating the absolute or relative amounts of BACE-1 in the sample (quantification). Methods for determining the levels of protein biomarkers, such as BACE-1, are known in the art and suitable methods are also disclosed herein through the examples of the present disclosure, for example, by use of an affinity reagent capable of specifically binding BACE-1.

Some aspects of the present disclosure, for example the second to sixth aspects, refer to the A/T/N (amyloid, Tau, neurodegeneration) classification system. In this sense, the A/T/N (amyloid, Tau, neurodegeneration) classification system refers to a research framework for AD diagnosis developed under the auspices of the National Institute on Aging and Alzheimer's Association (NIA-AA), which proposes that subjects may be categorised based on biomarker evidence of pathology, as disclosed in Jack et al., "NIA-AA Research Framework: Toward a biological definition of Alzheimer's disease", Alzheimer's & Dementia, 2018, vol. 14, issue 4, pp. 535-562, with the most recent update to the recommendations relating to the use of the A/T/N system disclosed in Jack et al., "Revised criteria for diagnosis and staging of Alzheimer's disease: Alzheimer's Association Workgroup", Alzheimer's & Dementia, 2024, vol. 20, issue 8, pp. 5143-5169. According to the A/T/N system, each individual is rated for the presence of pathological levels of (i) amyloid β-42 (determined as either the CSF concentration of Aβ42 or by using amyloid positron emission tomography-computed tomography (PET-CT); "A" biomarker category), (ii) hyperphosphorylated Tau (determined as either the CSF concentration of pTau181, Tau phosphorylated at position 181, or by using Tau PET-CT; "T" biomarker category), and (iii) neurodegeneration (assessed based on evidence of atrophy on structural magnetic resonance imaging (MRI), by 18F-fluordeoxyglucose PET-CT to evaluate changes in cerebral glucose metabolism, or by determining the concentration of total Tau (tTau) in CSF; "N" biomarker category), resulting in eight possible classification combinations.

Among other advantages, as disclosed in the relevant prior cited in the previous paragraph, classification of patients under the A/T/N system allows more personalised treatment recommendations. However, determining the A/T/N classification of a subject requires invasive testing, e.g. determining biomarker concentrations in the CSF, and resource-intensive protocols, in terms of the time, equipment, personnel training and financial investment required to carry out the necessary tests, e.g. PET-CT can only be carried out by highly-trained medical staff with access to costly, specialised equipment.

The skilled person is aware that, under the A/T/N system, the main relevant classification consists of the biomolecular-based classification of the amyloid-pathology by distinguishing "A+" from "A-" subjects, a differentiation that is crucial for deciding on the subsequent diagnosis, e.g. determining whether a subject is in the AD continuum or not according to the NIA-AA criteria, as well as the different medical management and treatment pathways suitable for each subject. For example, subject stratification for drug-based therapies targeting the amyloid pathology requires a positive amyloid (A+) classification.

The sixth, eighth and ninth aspects refer to "an earlier time point". In a particular embodiment according to these aspects, the earlier time point is at least 6 months previously, particularly at least 12 months previously.

The seventh and eight aspect refers to rate of memory decline. The rate of memory decline in a subject can be determined using methods known in the art, for example by determining repeated Mini-Mental State Examination (MMSE) or Montreal Cognitive Assessment (MoCA) scores, as disclosed, for example, in Miyakawa-Liu et al., "Rates of Cognitive Decline in 100 Patients With Alzheimer Disease", Ochsner J, 2022, vol. 22, issue 2, pp. 129-133. In a particular embodiment of the seventh or eighth aspects, the rate of memory decline is an annual rate of memory decline. In another particular embodiment, the rate of memory decline is a dementia-associated memory decline or Alzheimer's disease-associated memory decline.

The eighth aspect refers to "a change in the level of BACE-1". In a particular embodiment of the eighth aspect, the change in the level of BACE-1 in the subject is compared to a change in the level of BACE-1 in an age-matched control (i.e. cognitively normal) subject. The skilled person can establish, employing common general knowledge and the information disclosed herein, that the change in the level of BACE-1 in an age-matched control subject refers to the difference between the level of BACE-1 determined in an isolated sample from the age-matched control subject and the level of BACE-1 determined in an isolated sample from the same age-matched control subject at an earlier time point. The change in the level of BACE-1 may be a negative change (the level of BACE-1 is lower compared to the earlier time point), a positive change (the level of BACE-1 is higher compared to the earlier time point), or it may be no change (the level of BACE-1 is substantially equal compared to the earlier time point). In a more particular embodiment, when the change in the level of BACE-1 in the subject being tested using the methods of the present disclosure is higher than the change in the level of BACE-1 in an age-matched control subject, this is indicative that the rate of memory decline is an abnormal rate of memory decline.

The ninth aspect refers to a "pathological rate of memory decline". In a particular embodiment of the ninth aspect, the pathological rate of memory decline is a rate of memory decline that is at least 10% higher, particularly at least 15% higher, particularly at least 20% higher, particularly at least 25% higher, particularly at least 30% higher, particularly at least 35% higher, particularly at least 40% higher, particularly at least 45% higher, particularly at least 50% higher, than a rate of memory decline observed in a control subject over the same period of time. In another particular embodiment, the pathological rate of memory decline is a 2-point decrease, particularly a 3-point decrease, particularly a 4-point decrease, a 5-point decrease in Mini-Mental State Examination (MMSE) score. In a further particular embodiment of the ninth aspect, an increase in the level of BACE-1 in the isolated sample obtained from the subject, compared to the level of BACE-1 determined in an isolated sample obtained from the same subject at an earlier time point, is an increase of at least 5%, particularly at least 6%, particularly at least 7%, particularly at least 8%, particularly at least 9%, particularly at least 10%, particularly at least 12%, particularly at least 14%, particularly at least 16%, particularly at least 18%, particularly at least 20%, particularly at least 25%, particularly at least 30%, compared to the increase observed in an age-matched control subject over the same period of time.

The eleventh and twenty-second aspects refer to AD severity. In this sense, AD severity may be defined on the basis of the severity of the clinical symptoms, for example, as mild, moderate or severe AD, or, alternatively, by scoring of amyloid PET-CT scans, which can be acquired using methods known in the art, as summarised in Pemberton et al., "Quantification of amyloid PET for future clinical use: a state-of-the-art review", Eur J Nucl Med Mol Imaging, 2022, vol. 49, issue 10, pp. 3508-3528, or, alternatively, by Braak staging, wherein the Braak stage of a subject can be confirmed in brain tissue sections using methods known in the art, for example, as disclosed in Braak et al., "Staging of Alzheimer disease-associated neurofibrillary pathology using paraffin sections and immunocytochemistry", Acta Neuropathologica, 2006, vol. 112, issue 4, pp. 389-404.

In a particular embodiment of the present disclosure, the isolated sample obtained from the subject is a biological fluid selected from a list consisting of: blood, serum, plasma, lymph, cerebrospinal fluid, saliva, and urine. In a more particular embodiment, the isolated sample obtained from the subject is blood, serum, or plasma.

In a particular embodiment of the *in vitro* method according to the present disclosure, the population of EVs, in particular the population of BDEVs, comprises extracellular vesicles (EVs) with a diameter of 200 nm or below. In a more particular embodiment, the extracellular vesicles (EVs) with a diameter of 200 nm or below represent at least 80%, particularly at least 85%, more particularly at least 90%, of the population of EVs, particularly of the population of BDEVs, wherein the percentage represented by the EVs with a diameter of 200 nm or below is calculated as the number of EVs with a diameter of 200 nm or below in the population of EVs relative to the total number of EVs in the population, particularly is calculated as the number of EVs with a diameter of 200 nm or below in the population of BDEVs relative to the total number of BDEVs in the population. In another particular embodiment, the population of EVs, particularly the population of BDEVs, consists essentially of extracellular vesicles (EVs) with a diameter of 200 nm or below. In another particular embodiment, the population of BDEVs comprises brain-derived exosomes (BDEXs), more particularly comprises brain-derived exosomes that carry NLGN3. In yet another particular embodiment, the population of EVs, particularly the population of BDEVs, comprises extracellular vesicles (EVs) that pellet following ultracentrifugation for 1 hour at a speed of 100,000 x g. In this sense, "pellet" is understood to refer to the phenomenon wherein, under appropriate centrifugal force, EVs suspended in a liquid medium settle the bottom of a container used to hold a sample during centrifugation, such as a centrifuge tube, thus separating EVs from the remaining solution, the latter being commonly known as a "supernatant".

As used herein, the term "extracellular vesicles" (EVs) refers to vesicles enclosed by a lipid bilayer membrane, with sizes of EVs released by living cells typically ranging between 30 nm to 1 µm in size, which arise from various biogenesis pathways. As used herein, the term "brain-derived extracellular vesicles" or "BDEVs" refers to EVs derived from/secreted by/produced by/released by one or more brain cell types. EVs, including BDEVs, may be isolated from various biological fluids, including, but not limited to, blood, plasma, serum, lymph, urine and saliva.

EVs with a diameter of 200 nm or above, particularly 300 nm or above, may be referred to as microvesicles. Without being bound by any particular theory, microvesicles may arise from shedding of membrane-bound vesicles from the plasma membrane, in a process that entails the outer budding of the plasma membrane.

As used herein, the term "exosome" refers to lipid bilayer-enclosed extracellular vesicles with a diameter below 300 nm, particularly below 200 nm. Without being bound by any particular theory, exosomes are thought to arise as intraluminal vesicles within a cell's endocytic compartments and are secreted from the cell by fusion of late endocytic compartments, particularly the multi-vesicular body, with the plasma membrane. Exosomes are secreted by most cell types and carry a molecular cargo comprising proteins, nucleic acids, lipids and other metabolites. Although significant size overlap exists between exosomes and microvesicles, the two classes of EVs are characterised by different molecular cargoes, wherein molecules carried within EVs serve as a signature of their route of biogenesis.

A third class of EVs, known as "apoptotic bodies", are generated by blebbing of the plasma membrane that occurs during programmed cell death or apoptosis, but these are typically characterised by larger sizes, such as diameters between 1 µm to 5 µm.

EVs carry a protein cargo that is informative of their cell type or tissue of origin. In this sense, a protein expressed at significantly higher levels in the cell or tissue of origin, compared to the expression level of the protein across all cell types or tissues, or a protein whose expression is essentially specific (i.e. restricted) to the cell or tissue of origin, can serve as markers for populations of EVs that are secreted by, derived from different cell types or tissues. Proteins that display higher expression in a particular cell type or tissue may also be referred to as "enriched" in that particular cell type or tissue. In this sense, the expression level of protein X in a cell, tissue or biological sample is determined as µg protein X per mg total protein extracted from the cell, tissue or biological sample. Thus, populations of brain-derived EVs carry protein markers whose expression is higher/enriched in brain cells, particularly higher in neuronal cells, glial cells or both. Similarly, a population of neuron-derived EVs is understood to carry protein markers whose expression is higher/enriched in neurons. In particular, membrane proteins that are partly or fully exposed on the outside of EVs can serve as markers for populations of EVs, wherein said markers may be used to select, purify, isolate, concentrate or capture the populations of EVs secreted by different cell types or tissues.

In a particular embodiment of the present disclosure, the maker for the population of BDEVs is selected from a list of proteins consisting of: Neuroligin-3 (NLGN3), neural cell adhesion molecule 1 (NCAM1), Glutamate receptor 2 subunit (GRIA2), and sodium-potassium ATPase catalytic subunit alpha-3 (ATP1A3), V-set and transmembrane domain-containing protein 2B (VSTM2B), Neurocan core protein (NCAN), Neurexin-1-alpha (NRXN1), Neurexin-1-beta (NRXN1), Neurexin-2-alpha (NRXN2), Neurexin-2-beta (NRXN2), Neurexin-3-alpha (NRXN3), Neurexin-3-beta (NRXN3), Seizure protein 6 (SEZ6), Heparan Sulfate 6-O-Sulfotransferase 3 (HS6ST3), ATPase Plasma Membrane Ca2+ Transporting 3 (ATP2B3), Protocadherin Alpha-C2 (PCDHAC2), Netrin receptor DCC (DCC), Myelin-oligodendrocyte glycoprotein (MOG), Glutamate lonotropic Receptor AMPA Type Subunit 4 (GRIA4), Ferric Chelate Reductase 1 Like (FRRS1L), Transmembrane Protein 132D (TMEM132D), Ephrin type-A receptor 5 (EPHA5), ATPase Plasma Membrane Ca2+ Transporting 4 (ATP2B4), ATPase Plasma Membrane Ca2+ Transporting 2 (ATP2B2), G-protein coupled receptor 158 (GPR158), Contactin-associated protein-like 2 (CNTNAP2), Contactin-associated protein-like 5 (CNTNAP5), Neuronal pentraxin-1 (NPTX1), Neuronal pentraxin receptor (NPTXR), ST8 Alpha-N-Acetyl-Neuraminide Alpha-2,8-Sialyltransferase 3 (ST8SIA3), ST8 Alpha-N-Acetyl-Neuraminide Alpha-2,8-Sialyltransferase 5 (ST8SIA5), Potassium/sodium hyperpolarization-activated cyclic nucleotide-gated channel 1 (HCN1), Synaptotagmin-1 (SYT1), KIAA1549 Like (KIAA1549L), Cadherin 18 (CDH18), a GABA receptor subunit, Solute Carrier Family 39 Member 12 (SLC39A12), Multiple EGF Like Domains 10 (MEGF10), Dyslexia-associated protein KIAA0319 (KIAA0319), Adhesion G Protein-Coupled Receptor B1 (ADGRB1), G Protein-Coupled Receptor 37 (GPR37), G Protein-Coupled Receptor 37 Like 1 (GPR37L1), Carbonic Anhydrase 14 (CA14), and combinations thereof, particularly NLGN3. In an even more particular embodiment, the marker for the population of BDEVs is neuroligin-3 (NLGN3). Uniprot IDs of the human counterparts of the aforementioned proteins are provided in Table 1:

**Table 1: Uniprot ID, Protein name and Gene name corresponding to makers for the population of BDEVs according to a particular embodiment of the present disclosure**

| **Uniprot** | **ID Protein name** | **Gene name** |
|---|---|---|
| A6NLU5 | V-set and transmembrane domain-containing protein 2B | VSTM2B |
| O14514 | Adhesion G protein-coupled receptor B1 | ADGRB1 |
| O14594 | Neurocan core protein | NCAN |
| O15354 | G-protein coupled receptor 37 | GPR37 |
| O15466 | ST8 alpha-N-acetyl-neuraminide alpha-2,8-sialyltransferase 5 | ST8SIA5 |
| O43173 | ST8 alpha-N-acetyl-neuraminide alpha-2,8-sialyltransferase 3 | ST8SIA3 |
| O60741 | Potassium/sodium hyperpolarization-activated cyclic nucleotide-gated channel 1 | HCN1 |
| O60883 | G-protein coupled receptor 37-like 1 | GPR37L1 |
| O95502 | Neuronal pentraxin receptor | NPTXR |
| P13591 | Neural cell adhesion molecule 1 | NCAM1 |
| P13637 | Sodium/potassium-transporting ATPase subunit alpha-3 | ATP1A3 |
| P21579 | Synaptotagmin-1 | SYT1 |
| P23634 | Plasma membrane calcium-transporting ATPase 4 | ATP2B4 |
| P42262 | Glutamate receptor 2 | GRIA2 |
| P43146 | Netrin receptor DCC | DCC |
| P48058 | Glutamate receptor 4 | GRIA4 |
| P54756 | Ephrin type-A receptor 5 | EPHA5 |
| P58400 | Neurexin-1-beta | NRXN1 |
| P58401 | Neurexin-2-beta | NRXN2 |
| Q01814 | Plasma membrane calcium-transporting ATPase 2 | ATP2B2 |
| Q13634 | Cadherin-18 | CDH18 |
| Q14C87 | Transmembrane protein 132D | TMEM132D |
| Q15818 | Neuronal pentraxin-1 | NPTX1 |
| Q16653 | Myelin-oligodendrocyte glycoprotein | MOG |
| Q16720 | Plasma membrane calcium-transporting ATPase 3 | ATP2B3 |
| Q504Y0 | Zinc transporter ZIP12 | SLC39A12 |
| Q53EL9 | Seizure protein 6 homolog | SEZ6 |
| Q5T848 | Metabotropic glycine receptor | GPR158 |
| Q5W43 | Dyslexia-associated protein KIAA0319 | KIAA0319 |
| Q6ZVL6 | protein KIAA1549L | KIAA1549L |
| Q8IZP7 | Heparan-sulfate 6-O-sulfotransferase 3 | HS6ST3 |
| Q8WYK1 | Contactin-associated protein-like 5 | CNTNAP5 |
| Q96KG7 | Multiple epidermal growth factor-like domains protein 10 | MEGF10 |
| Q9HDB5 | Neurexin-3-beta | NRXN3 |
| Q9NZ94 | Neuroligin-3 | NLGN3 |
| Q9P0K9 | Ferric-chelate reductase 1-like protein | FRRS1L |
| Q9P2S2 | Neurexin-2-alpha | NRXN2 |
| Q9UHC6 | Contactin-associated protein-like 2 | CNTNAP2 |
| Q9ULB1 | Neurexin-1-alpha | NRXN1 |
| Q9ULX7 | Carbonic anhydrase 14 | CA14 |
| Q9Y4C0 | Neurexin-3-alpha | NRXN3 |
| Q9Y5I4 | Protocadherin alpha-C2 (PCDH-alpha-C2) | PCDHAC2 |

Size distribution of EVs may be determined using methods known in the art, for example, by nanoparticle tracking analysis (NTA) as disclosed in Comfort et al., "Nanoparticle Tracking Analysis for the Quantification and Size Determination of Extracellular Vesicles", 2021, Journal of Visualized Experiments, vol. 169, pp: e62447, or by Dynamic Light Scattering (DLS) as disclosed in Khan et al., "Determining the Size Distribution and Integrity of Extracellular Vesicles by Dynamic Light Scattering", 2022, Methods in Molecular Biology, vol. 2413, pp. 165-175.

The molecular cargo of exosomes and other EVs may be indicative of the status of the cell of origin, including of ongoing pathological processes in the cell of origin. Thus, without wishing to be bound by a specific theory or mechanism of action, assessing molecular cargoes, such as determining the level of BACE-1 according to the present disclosure, in BDEVs that cross the blood brain barrier (BBB), into the systemic circulation or other peripheral biological fluids, enables diagnosing, and/or monitoring the status of, neurodegenerative pathologies affecting central nervous system (CNS) neurons, particularly pathological memory decline, MCI, AD, AD-associated pathological changes, or AD-associated dementia, with high specificity and improved sensitivity by means of a rapid, non-invasive, cost-efficient test, such as a blood test, without the need for performing a lumbar puncture, a brain tissue biopsy or other invasive testing, and by using equipment or devices that require lower financial investment and that can be operated in a general care medical setting. The methods disclosed herein are further compatible with large-scale applications, including robust and accurate assays and systems amenable for commercial, clinical-grade and high-throughput automation.

In a particular embodiment of the *in vitro* method according to the present disclosure, the population of BDEVs comprises neuron-derived EVs, particularly neuron-derived EVs that carry neuroligin-3 (NLGN3). In a more particular embodiment, the number of neuron-derived EVs, particularly the number of neuron-derived EVs that carry neuroligin-3 (NLGN3), represents at least 80%, particularly at least 85%, more particularly at least 90% of the total number of EVs in the population of BDEVs. In an even more particular embodiment, the population of BDEVs consists essentially of neuron-derived EVs, particularly consists essentially of neuron-derived EVs that carry neuroligin-3 (NLGN3). In another even more particular embodiment, the neuron-derived EVs are neuron-derived exosomes, particularly neuron-derived exosomes that carry neuroligin-3 (NLGN3).

In another particular embodiment of the *in vitro* method according to the present disclosure, the population of BDEVs comprises glial-derived EVs, particularly glial-derived EVs that carry neuroligin-3 (NLGN3). In another particular embodiment, the number of glial-derived EVs, particularly the number of glial-derived EVs that carry neuroligin-3 (NLGN3), represents at least 80%, particularly at least 85%, more particularly at least 90% of the total number of EVs in the population of BDEVs. In a more particular embodiment, the glial-derived EVs are derived from a brain cell type selected from a list consisting of: astrocytes, oligodendrocytes, ependymal cells, microglia and combinations thereof. In another more particular embodiment, the glial-derived EVs are astrocyte-derived EVs. In an alternative more particular embodiment, the glial-derived EVs are not astrocyte-derived EVs. In a yet more particular embodiment, the population of BDEVs consists essentially of glial-derived EVs, particularly consists essentially of glial-derived EVs that carry neuroligin-3 (NLGN3). In a further more particular embodiment, the glial-derived EVs are glial-derived exosomes, particularly glial-derived exosomes that carry neuroligin-3 (NLGN3).

In a particular embodiment of the *in vitro* method according to the present disclosure, the step of determining the level of BACE-1 comprised in the population of brain-derived extracellular vesicles (BDEVs) comprises contacting the population of BDEVs within the isolated sample with an affinity reagent capable of specifically binding BACE-1. In a more particular embodiment, the affinity reagent capable of specifically binding BACE-1 is an anti-BACE-1 antibody or an anti-BACE-1 functional antibody fragment. In another more particular embodiment, the affinity reagent capable of specifically binding BACE-1 is capable of binding the extracellular domain of BACE-1. In a further more particular embodiment, determining the level of BACE-1 comprised in the population of BDEVs comprises determining the level of BACE-1 comprised in intact EVs. In this sense, "intact EVs" refers to EVs that have not been exposed to treatments, conditions or methods that permeabilise, lyse, disrupt or damage EV membranes, or that solubilise membrane proteins. In other words, intact EVs, as used herein, refers to EVs that retain membrane integrity. In an alternative more particular embodiment, determining the level of BACE-1 comprised in the population of BDEVs may comprise a step of lysing EVs, optionally, following pretreatment of EVs with proteinase K or trypsin. The skilled person is aware that proteinase K or trypsin may be employed to degrade exposed proteins or protein domains, such as proteins that are not carried in EVs as well as domains of EV-cargo proteins that are exposed externally on EVs.

As used herein, "affinity reagent" refers to a compound or molecule that has the ability to specifically bind to a given target molecule. Suitable affinity reagents that may be used in the context of the present disclosure include, but are not limited to, antibodies, functional antibody fragments, receptors, ligands (including small molecules and interacting proteins), monobodies, designed ankyrin repeat proteins (DARPins) and aptamers (including nucleic acid aptamers and peptide aptamers). In this sense, the skilled person understands that "ligands" is a term used to refer to molecules that specifically interact with their target proteins, e.g. with a brain-enriched membrane protein that may act as a marker for a population of BDEVs. In this sense, ligands may alternatively be referred to as interacting molecules. A ligand may be, for example, a small molecule, or alternatively, a protein or protein fragment. In the context of affinity reagents directed to membrane proteins, whose expression is enriched in brain cell types, and which are located at the synapse as target molecules (e.g. NLGN3 ligands), ligands of such synaptic proteins may be defined as the membrane proteins located on the opposing part of the synapse (e.g. presynaptic in the case of a postsynaptic target, and vice a versa) and interact with said target as part of the synaptic structure. For example, NLGN3 is known to interact with NRXN1, NRXN2, NRXN3, PTPRD, PIC3R1 and PIC3R2. One or more of such proteins, or one or more fragments thereof which mediate the interaction of such proteins with NLGN3, can be used as an affinity reagent that is capable of specifically binding to NLGN3. Furthermore, the skilled person understands that "receptors" refers to macromolecules or macromolecular complexes, which may typically be a protein or a protein complex, optionally in combination with cofactors, that bind to a given target molecule. In biological systems, receptors are typically found at cell membranes where they act to transduce signals to the cell. Ligands and receptors suitable for use in the context of the present disclosure may comprise modifications that improve their stability or affinity using methods known in the art. For example, the ligands or receptors may comprise the wildtype amino acid sequence or an improved amino acid sequence, wherein the improved amino acid sequence is an amino acid sequence that provides desirable binding characteristics between the ligand or receptor and the target protein, such as, but not limited to, higher binding affinity or improved specificity. The improved amino acid sequence may be determined through methods known in the art, including, but not limited to, artificial evolution, random or direct maturation, and substitution with non-natural amino acids.

In the context of the present disclosure, the expressions "specifically binds", "specifically recognises", or, alternatively, affinity reagent "specific to"/"capable of specifically binding" a given target molecule, refer to an affinity reagent that binds to a target molecule (such as a biomarker comprised in an exosome, for example BACE-1, or a marker present on the surface of an exosome, for example NLGN3) with at least 5-fold greater affinity, as compared to a non-target molecule. In a particular embodiment, the affinity reagent that specifically binds to the target molecule, binds the target molecule with at least 7-fold, particularly with at least 10-fold, more particularly with at least 15-fold, even more particularly with at least 20-fold, most particularly with at least 50-fold greater affinity, as compared to a non-target molecule.

The skilled person understands that the term "antibody" refers to a full-length, Y-shaped protein belonging to the immunoglobulin superfamily that has the ability to bind a target molecule, particularly a target protein, known as an antigen. The term "antigen" as used herein is a molecule that an antibody or a functional antibody fragment binds to. An antigen is typically capable of inducing an immune response in an animal, resulting in the production of an antibody capable of binding to one or more epitopes comprised in the antigen. The skilled person understands that an "epitope" refers to the portion, or portions, of the antigen that the antigen-binding region of the antibody or the functional antibody fragment binds to and whose presence is, or are, necessary in order for the antibody to bind to the target antigen. Antibodies include, for example, polyclonal antibodies (pAbs), monoclonal antibodies (mAbs), chimeric antibodies, recombinant antibodies and engineered antibodies.

In the context of the present disclosure, it would be apparent to the skilled person that the affinity reagent need not necessarily be a full-length antibody but may be a functional antibody fragment or any other molecule that is able to specifically bind to the target molecule of interest (for example BACE-1 or NLGN3). A "functional antibody fragment" refers to a fragment or portion of an antibody, wherein said fragment or portion is capable of specifically binding the target antigen, with an affinity similar to that of the full-length antibody from which it is derived. Thus, "functional antibody fragments" are macromolecules comprising whole, or essentially whole, variable regions of both light and heavy chains (forming an antigen-binding portion) of an antibody. Functional antibody fragments suitable for use as affinity reagents in the context of the present disclosure include but are not limited to Fab fragments, single-chain fragment variables (scFvs), minibodies, diabodies, and single-domain antibodies (sdAbs).

Methods of generating affinity reagents, including, but not limited to, monoclonal antibodies, polyclonal antibodies, functional antibody fragments and aptamers, are well known in the art. Moreover, monoclonal and polyclonal antibodies suitable for use in the context of the present disclosure that are capable of specifically binding BACE-1 or EV markers, including BDEV markers, such as NLGN3 are commercially available.

In another particular embodiment of the *in vitro* method according to the present disclosure, the step of determining the level of BACE-1 comprised in brain-derived extracellular vesicles is performed on a point-of-care (PoC) device, particularly on a PoC device that comprises a microfluidic chip. In a more particular embodiment, the PoC device comprises one or more sensors selected from a list consisting of: a chemical sensor, an electrical sensor, an electrochemical sensor, a paramagnetic sensor, and an optical sensor.

In a particular embodiment of the present disclosure, the *in vitro* method comprises the following steps: (i) capturing of the population of brain-derived extracellular vesicles (BDEVs) comprised within the isolated sample; and (ii) determining the level of BACE-1 comprised in the population of brain-derived extracellular vesicles (BDEVs) captured in step (i). In a more particular embodiment, step (i) is carried out using an affinity reagent capable of specifically binding a marker for the population of BDEVs, wherein the marker for the population of BDEVs is a brain-enriched membrane protein. In a further more particular embodiment, the marker for the population of BDEVs is a membrane protein enriched in a brain cell type, particularly wherein the brain cell type is a neuron, a glia, or a combination thereof. In an even more particular embodiment, step (i) is carried out using an affinity reagent capable of specifically binding neuroligin-3 (NLGN3), more particularly is carried out using an anti-NLGN3 antibody or an anti-NLGN3 functional antibody fragment. In an alternative even more particular embodiment, the affinity reagent is an aptamer capable of specifically binding to a marker for the population of BDEVs, particularly a nucleic acid aptamer or a peptide aptamer, more particularly an aptamer capable of specifically binding NLGN3. In another particular embodiment, the affinity reagent is a ligand that interacts with the marker for the population of BDEVs, particularly a ligand that interacts with NLGN3. In a further particular embodiment, the affinity reagent is a protein that interacts with the marker for the population of BDEVs, or is a fragment thereof which mediates the interaction of the protein with the marker for the population of BDEVs, particularly wherein the marker for the population of BDEVs is NLGN3. In another more particular embodiment, step (i) comprises capturing of the population of BDEVs comprised within the isolated sample onto an insoluble support. In a yet more particular embodiment, the insoluble support comprises magnetic particles, particularly magnetic beads. In yet another more particular embodiment, step (i) is carried out by a method comprising a magneto-actuated assay. In a more particular embodiment, the magneto-actuated assay comprises contacting the isolated sample obtained from the subject with an affinity reagent capable of specifically binding a marker for the population of BDEVs, wherein the marker for the population of BDEVs is a membrane protein enriched in a brain cell type, particularly wherein the marker for the population of BDEVs is neuroligin-3 (NLGN3). In a more particular embodiment, the affinity reagent capable of specifically binding the marker for the population of BDEVs is immobilised on an insoluble support. In another more particular embodiment, the magneto-actuated assay is a magneto-actuated immunoassay wherein the affinity reagent capable of specifically binding the marker for the population of BDEVs is an antibody or functional antibody fragment, particularly an anti-NLGN3 antibody or an anti-NLGN3 functional antibody fragment. In an even more particular embodiment, the insoluble support comprises a plurality of magnetic particles, particularly magnetic beads. As used herein, a "magneto-actuated assay" refers to an assay that comprises the separation of a target molecule and/or EV from its physical environment under magnetic actuation, through magnetic separation.

Furthermore, in a particular embodiment of the twenty-eighth aspect, the *in vitro* method comprises the following steps: (i) capturing of the population of extracellular vesicles (EVs) that carry neuroligin-3 (NLGN3) comprised in an isolated sample obtained from the subject; and (ii) determining the level of BACE-1 comprised in the population of extracellular vesicles (EVs) captured in step (i). In a more particular embodiment, step (i) is carried out using an affinity reagent capable of specifically binding neuroligin-3 (NLGN3), more particularly is carried out using an anti-NLGN3 antibody or an anti-NLGN3 functional antibody fragment. In another more particular embodiment, step (i) is carried out using an aptamer capable of specifically binding neuroligin-3 (NLGN3). In a yet more particular embodiment, step (i) comprises capturing of the population of EVs comprised in the isolated sample onto an insoluble support, particularly wherein the insoluble support comprises magnetic particles, more particularly wherein the insoluble support comprises magnetic beads. In an even more particular embodiment, the affinity reagent capable of specifically binding NLGN3 is immobilised on an insoluble support.

The skilled person is aware that populations of EVs may be isolated, purified, concentrated, pre-concentrated, enriched or captured from biological samples based on their physicochemical characteristics, using methods known in the art, for example, as disclosed in Zhang et al., "Comprehensive isolation of extracellular vesicles and nanoparticles", Nature Protocols, 2023, vol. 18, pp. 1462-1487. As used herein, "isolation", "purification", "enrichment", "concentration" or "capturing" of EVs refers to their physical separation from their environment (e.g. biofluid sample), to obtain a substantially pure or an enriched EV population. Following isolation, purification, enrichment, concentration or capturing of the population of BDEVs present in the isolated sample, an enriched sample may be obtained, wherein the concentration of BDEVs in the enriched sample is greater than the concentration of BDEVs in the isolated sample.

Populations of EVs may additionally, or alternatively, be captured, optionally onto an insoluble support, using molecules present on their surface. Such a surface molecule may serve as a marker for a population of EVs and may be recognised by affinity reagents capable of specifically binding said surface molecule. In this sense, "capturing" EVs refers to a process whereby the physical separation of EVs from their environment comprises attaching EVs to and/or retaining EVs on a physical support. For example, prior to determining the level of BACE-1 according to the present disclosure, a population of EVs, particularly a population of BDEVs, comprised in the biological sample may be captured using protein markers, in particular protein makers for specific populations of EVs or EV sub-types, such as exosomes. The skilled person is familiar with exosome-specific or exosome-selective marker proteins, which include: tetraspanin proteins - such as CD9, CD63, and CD81 -, ALG 2-interacting protein X (ALlX), tumour susceptibility gene 101 protein (TSG101) and ESCRT proteins, as well as proteins involved in cell adhesion and signalling, cytoskeletal structures, lipid rafts or membrane trafficking. Additionally, or alternatively, EVs comprised in the isolated sample may be captured using markers for populations of EVs derived from specific organs and/or cell types of interest, in particular markers of brain-derived EVs (BDEVs). Suitable markers of BDEVs for use in the context of the present disclosure comprise membrane proteins whose expression is enriched in a brain cell type, particularly in a brain cell type selected from: neurons, astrocytes, oligodendrocytes, ependymal cells, microglia, and combinations thereof. NLGN3 is a neuron cell-surface marker localised at subsets of both excitatory and inhibitory synapses. NLGN3 is expressed exclusively at brain level, in particular in the hippocampus, which is one of brain areas first affected by AD. This makes NLGN3 a particularly good candidate marker to concentrate neuron-derived EVs for early AD detection.

As detailed above, in more particular embodiments of the *in vitro* method of the present disclosure, the method comprises a step of capturing of the population of extracellular vesicles (EVs), particularly capturing of the population of brain-derived extracellular vesicles (BDEVs), comprised within the isolated sample on an insoluble support. In further more particular embodiments, the step of capturing of the population of EVs, particularly capturing of the population of BDEVs, is carried out using an affinity reagent immobilised on the insoluble support. In even more particular embodiments, the affinity reagent is immobilised on the insoluble support via a toluenesulfonyl group. As used herein, the term "immobilised" denotes that the affinity reagent is attached to the insoluble support by covalent conjugation or other forms of chemical linkage that are stable under the conditions used in the methods of the disclosure, such that isolation, enrichment, capture or identification of EVs bound by the affinity reagent is possible. The skilled person is familiar with methods suitable for immobilising affinity reagents on different types of suitable insoluble support. The skilled person is further aware of suitable methods for immobilising an affinity reagent on an insoluble support, for example, through direct chemical conjugation, through a streptavidin-biotin complex, or through linkers, including cleavable linkers. Suitable linkers for use in the context of the present disclosure are known in the art.

Examples of suitable insoluble support in the context of the present disclosure include, but are not limited to, particles (in particular beads), including, in particular, magnetic particles (more in particular, magnetic beads), nanoparticles, columns, plates, microfluidic structures, liposomes, or combinations thereof. In an alternative more particular embodiment, the insoluble support may comprise a column, particularly an affinity column. In another alternative particular embodiment, the insoluble support may comprise a surface of an assay plate, assay tube or assay column. In this sense, the assay plate can be of different sizes including, but not limited to, 384, 96, 24, 12 and 6-well plates.

As used herein, "particle" is understood, as generally in the art, as a small, localised object to which can be ascribed several physical or chemical properties, such as shape, volume, density, or mass. They vary greatly in size, shape, or quantity, from subatomic particles like the electron, to microscopic particles like atoms and molecules, to macroscopic particles like powders and other granular materials. Anything that is composed of particles may be referred to as being particulate. In the context of the present disclosure, the insoluble support may particularly comprise a plurality of nanoparticles and/or microparticles. Both nanoparticles and microparticles may be of different composition, such as, but not limited to, metal, organic, metalorganic, polymeric, quantum dots, or carbon structures. Nanoparticles and microparticles have at least two dimensions in the nanoscale or microscale, respectively, preferably all three dimensions in the nanoscale or microscale, and may have different shapes and sizes, for example spheres, rods, discs, tubes and hemispheres.

As used herein, the term "magnetic particle" refers to a nano- or micro-sized particle that is attracted to or repelled by a magnetic field gradient or has a non-zero magnetic susceptibility. Magnetic particles commonly comprise magnetic elements such as iron, nickel and cobalt and their chemical compounds. Magnetic particles, including magnetic beads, suitable for use in the context of the present disclosure are well-known in the art and are also available commercially, either with or without functional groups capable of forming chemical links with affinity reagents. The magnetic particle may be a paramagnetic particle or a super-paramagnetic particle. In this sense, "super-paramagnetic particles" refers to particles, with core diameters typically ranging between 10 to 300 nm, that exhibit magnetic behaviour only in the presence of an external magnetic field, ensuring that they do not attract each other outside of a magnetic field, thus avoiding unwanted clumping, which enables their use on automated platforms. Upon removal of the magnetic field, super-paramagnetic particles lose magnetic remanence and become dispersible essentially immediately. Super-paramagnetic particles may comprise magnetite (Fe3O4) and, optionally, one or more polymers. The surface of superparamagnetic particles may be coated with functional groups or elements, including but not limited to toluenesulfonyl groups (also known as a tosyl groups), that enable chemical conjugation/linking of the affinity reagent to the bead.

In embodiments of the present disclosure, the affinity reagent capable of specifically binding BACE-1 may be chemically modified to enable its downstream detection, for example, by coupling with biotin. Binding of the affinity reagent to BACE-1 may be evaluated or measured using methods known in the art, including but not limited to: (i) by using a labelled second affinity reagent (for example, a secondary antibody or streptavidin) that, in turn, is capable of specifically binding to the affinity reagent capable of specifically binding BACE-1, and/or (ii) by directly labelling the affinity reagent capable of specifically binding BACE-1, for example, with a fluorophore moiety, chromophore moiety, enzyme moiety, or radioisotope. In this sense, suitable enzyme moieties that may be used to label the affinity reagent capable of specifically binding BACE-1 include, but are not limited to, horseradish peroxidase (HRP), horseradish galactosidase and alkaline phosphatase. In a particular embodiment, determining the level of BACE-1 is carried out using a second affinity reagent, particularly a second affinity reagent labelled with a biotin-binding molecule conjugated with an HRP polymer, wherein the second affinity reagent is capable of specifically binding to the affinity reagent capable of specifically binding BACE-1. The skilled person is aware that the use of a second affinity reagent labelled with a biotin-binding molecule, such as streptavidin, conjugated with an HRP polymer, allows amplification of the signal to enable more sensitive detection. In particular, the second affinity reagent may be labelled with Streptavidin Poly-HRP. Platforms for amperometric electrochemical signal detection, chemiluminescent signal detection, electrochemiluminescent (ECL) signal detection or fluorescent signal detection are well known in the art and may readily be used for evaluating or measuring the binding of the affinity reagent to BACE-1, which may be used to determine the level of BACE-1.

Thus, in particular embodiments of the present disclosure, the method comprises quantifying a relative or absolute amount of an *in vitro* complex comprising an affinity reagent, such as an antibody or a functional antibody fragment, bound to BACE-1 comprised in the population of EVs, particularly in the population of BDEVs. In a more particular embodiment, the *in vitro* complex comprises a labelled affinity reagent, wherein the labelled affinity reagent is conjugated with a moiety selected from a list consisting of: biotin, horse radish peroxidase (HRP), alkaline phosphatase (AP), glucose oxidase, a fluorescent moiety, a phosphorescent moiety, an oligonucleotide, or combinations thereof.

In particular embodiments of the present disclosure, determining the level of BACE-1 is carried out by a method selected from the group consisting of: mass spectrometry, enzymatic analysis, colorimetric analysis, fluorometric analysis, luminometric analysis, turbidimetric analysis, electrochemical analysis, and combinations thereof, particularly electrochemical analysis. In a further particular embodiment, determining the level of BACE-1 is caried out with an assay using an affinity reagent. In a more particular embodiment, the assay is an immunoassay, particularly wherein the immunoassay is selected from a list consisting of: a dipstick assay, an enzyme-linked immunosorbent assay (ELISA), an antibody array, an antibody chip, a lateral flow test, a multiplex bead immunoassay, and combinations thereof. In this sense, determining the level of BACE-1 may comprise contacting the population of BDEVs with an antibody or a functional antibody fragment capable of specifically binding to BACE-1.

In particular, determining the level of BACE-1 in the context of the present disclosure may be carried out by a microfluidic assay, such as a lateral flow assay. The lateral flow test may be implemented on a miniaturised scale and is based on similar principles to a classical or magneto-actuated enzyme-linked immunosorbent assay (ELISA), which the skilled person is familiar with.

In more particular embodiments of the present disclosure, determining the level of BACE-1 comprised in the population of BDEVs in the isolated sample is carried out directly on the isolated sample, particularly on a non-diluted or a diluted plasma sample. In further more particular embodiments, the isolated sample is contacted simultaneously with a first affinity reagent capable of specifically binding a marker for a population of BDEVs and with a second affinity reagent capable of specifically binding BACE-1 comprised in the population of BDEVs.

In a fourteenth aspect, the present disclosure relates to a method of treating Alzheimer's disease or Alzheimer's disease-associated dementia. In this sense, the medical regimen may comprise administering a pharmacological agent selected from a list consisting of: an acetylcholine esterase inhibitor, a N-methyl-D-aspartate (NMDA) receptor antagonist, an anti-Tau antibody, a Tau vaccine, an anti-Aβ antibody, an orexin-receptor antagonist, an atypical antipsychotic, an adenosine antagonist, a synthetic cannabinoid, a GLP-1 agonist, or combinations thereof. The person skilled in the art is able to select pharmacological agents belonging to each category that are suitable for administration to a subject in the context of the methods of the present disclosure. Suitable pharmacological agents and medical regimens for the treatment of Alzheimer's disease or for the treatment of Alzheimer's disease-associated dementia that may be administered to the subject are known in the art, for example, but not limited to, as summarised in Cummins et al., "Alzheimer's disease drug development pipeline: 2024", Alzheimer's & Dementia, 2024, vol. 10, pp: e12465, or as summarised in Huang et al., "Clinical trials of new drugs for Alzheimer disease: a 2020-2023 update", Journal of Biomedical Sciences, 2023, vol. 30, issue 1, pp: 83. For example, the cholinesterase inhibitor may be selected from the group consisting of: Donepezil, Rivastigmine, Galantamine and Tacrine, particularly Donepezil or Galantamine. Suitable N-methyl-D-aspartate (NMDA) receptor antagonists for administration to a subject in the context of the present disclosure include, but are not limited to, Memantine, AVP-786, and AXS-05. The NMDA receptor agonist may particularly be Memantine. Suitable anti-Aβ antibodies for use in the treatment of AD and AD symptomatology include, but are not limited to, Lecanemab, Remternetug, Buntanetap, Solanezumab, Donanemab and Aducanumab. Atypical antipsychotics suitable for administration to a subject in the context of the present disclosure include, but are not limited to, Brexpriprazole. Orexin receptor antagonists suitable for administration to a subject in the context of the present disclosure include, but are not limited to, Daridorexant, Suvorexant, and Lemborexant. GLP-1 agonists suitable for administration to a subject in the context of the present disclosure include, but are not limited to, Semaglutide. A "Tau vaccine" is understood to refer to a vaccine comprising a peptide derived from the Tau protein, also known as the microtubule-associated Tau protein, encoded by the MAPT gene. In the context of the present disclosure, the medical regimen for the treatment of Alzheimer's disease or for the treatment of Alzheimer's disease-associated dementia to be administered to the subject may particularly comprise administration of a NMDA receptor antagonist, more particularly Memantine, or, alternatively, administration of a combination of an acetylcholine esterase inhibitor and a NMDA receptor antagonist, more particularly of a combination of Donepezil and Memantine.

The fifteenth aspect of the present disclosure relates to a method of deciding or recommending whether to initiate a medical regimen in a subject suspected of suffering from Alzheimer's disease or of being at risk of developing Alzheimer's disease. In the context of the present disclosure, "at risk of developing Alzheimer's disease" includes genetic risk, which may comprise single nucleotide polymorphisms or mutations known to cause or increase risk of AD, such as, example, the ε4 allele of the APOE gene, or mutations in *PSEN1, PSEN2* or *APP.* Additionally, or alternatively, a subject may be deemed at risk of developing AD based on a genetic risk score, such as a polygenic risk score (PRS) or a polygenic hazard score (PHS). Methods to determine a subject's PRS and/or PHS in relation to risk of developing AD are known in the art, for example, as disclosed in Clark et al., "Polygenic Risk Scores in Alzheimer's Disease Genetics: Methodology, Applications, Inclusion, and Diversity", Journal of Alzheimer's Disease, 2022, vol. 89, issue 1, pp. 1-12, or in Leonenko et al., "Identifying individuals with high risk of Alzheimer's disease using polygenic risk scores", Nature Communications, 2021, vol. 12, article number 4506. Also considered to be "at risk of developing Alzheimer's disease" are subjects with specific combinations of risk factors that may increase likelihood of developing AD, including, but not limited to, over 65 years of age, type 2 diabetes, cardiovascular disease, a family history of AD, genetic risk factors, and combinations thereof. In a particular embodiment of the present disclosure, the subject carries a genetic mutation known to be correlated with an increased risk of developing AD. In a more particular embodiment, the subject is a carrier of the ε4 or ε3 allele of the APOE gene. In another more particular embodiment, the subject carries a mutation in at least one gene selected from a list consisting of amyloid precursor protein gene (APP), presenilin 1 gene (PSEN1), and presenilin 2 gene (PSEN2).

In more particular embodiments of the present disclosure, the subject may be a subject with mild cognitive impairment.

The sixteenth aspect of the present disclosure relates to a method of deciding or recommending whether to initiate a medical regimen in a subject and the seventeenth aspect provides an *in vitro* method of determining the level of brain-derived β-secretase 1 protein (BACE-1).

All particular embodiments disclosed above are also embodiments of the fourteenth to the seventeenth aspects.

In a particular embodiment of the *in vitro* method according to the present disclosure, the subject is a human. In a more particular embodiment, the human is 65-years old or older. In another more particular embodiment, the human is 60-years old or younger. In another particular embodiment, the subject is suspected of having AD or of being predisposed to develop AD. In this sense, the subject may be suspected of having AD based on the presence of one or more symptoms or disease manifestations of AD, including, but not limited to, loss of short-term memory, impaired reasoning, difficulty handling complex tasks, poor judgment, language dysfunction, and visuospatial dysfunction. Additionally, or alternatively, the subject may be suspected of having AD based on the presence of dementia. Additionally, or alternatively, the subject may be suspected of having AD, or of being predisposed to developing AD, based on the presence of one or more risk factors associated with AD. As used herein, "predisposed" to a disease such as a neurodegenerative disease may refer to a genetic, familial or chemically-induced predisposition, e.g. as described above. A subject suspected of being predisposed to AD may or may not display symptoms or disease manifestations.

The eighteenth aspect of the present disclosure relates to a kit, while the nineteenth aspect relates to a device. In a particular embodiment, the kit or device according to the present disclosure, further comprises one or more sensors selected from the group consisting of: a chemical sensor, an electrical sensor, an electrochemical sensor, a paramagnetic sensor, and an optical sensor. In a more particular embodiment, the kit or device comprises an electrochemical sensor.

Devices of suitable construction for use in the context of the present disclosure are known in the art. In particular, the device of the nineteenth aspect may be a device as disclosed in WO2023006817A1. The entire contents of WO2023006817A1 are herein incorporated by reference. Thus, in a particular embodiment, the device of the nineteenth aspect further comprises:
- an electrode (92, 102);
- a first cover (41) comprising
- a chamber (11, 411, 91) comprising
- a first opening (111, 911) for adding magnetic particles and liquids (45) into the chamber (11, 411, 91),
- and a second opening (112) for letting the magnetic particles and liquids contact the electrode (92, 102) placed in the device adjacent to the chamber (11, 411, 91);
- and a sealing element (12, 412);
- a second cover (42) fitting the first cover (41), the second cover (42) comprising
- a liquid absorbing pad (13, 421) adjacent to the chamber (11, 411, 91);
- a liquid exit (15, 43) for letting the liquid exit the chamber (11, 411, 91);
- an opening assembly (14, 46) for shifting the device between a closed position and an open position, the opening assembly (14, 46) comprising
- a first cooperating mechanism (461) and a second cooperating mechanism (462) wherein the first cover (41) comprises the first cooperating mechanism (461) and the second cover (42) comprises the second cooperating mechanism (462); wherein:
- the sealing element (12, 412) is for sealing the liquid exit (15, 43);
- the liquid absorbing pad (13, 421) faces at least part of the liquid exit (15, 43) of the chamber (11, 411, 91) in the open position of the device; and wherein:
- in the closed position the sealing element (12, 412) covers the liquid exit (15, 43) such that a liquid is prevented from flowing out of the chamber (11, 411, 91) through the liquid exit (15, 43);
- and in the open position:
- at least part of the liquid exit (15, 43) is uncovered by the sealing element (12, 412) and - the device comprises a fluidic path (16, 44) between the chamber (11, 411, 91) and the liquid absorbing pad (13, 421) through the uncovered part of the liquid exit (15, 43). In a particular embodiment of the kit according to the eighteenth aspect or of the device according to the nineteenth aspect, the means for capturing a population of BDEVs comprises an affinity reagent capable of specifically binding to a marker for the population of BDEVs. In a more particular embodiment, the affinity reagent capable of specifically binding to the marker for the population of BDEVs comprises an anti-NLGN3 antibody or anti-NLGN3 functional antibody fragment. In an even more particular embodiment, the anti-NLGN3 antibody or anti-NLGN3 functional antibody fragment is coupled to an insoluble support, particularly to magnetic particles, more particularly to magnetic beads.

In a particular embodiment of the kit according to the eighteenth aspect or of the device according to the nineteenth aspect, the means for detecting the presence and/or for determining the level of BACE-1 protein comprises an affinity reagent capable of specifically binding to BACE-1, particularly comprises an anti-BACE-1 antibody or anti-BACE-1 functional antibody fragment. The affinity reagent capable of specifically binding to BACE-1 may be further labelled to enable its downstream detection as detailed above, or a second affinity reagent may be employed to evaluate the binding to BACE-1 of the affinity reagent capable of specifically binding to BACE-1, as also detailed above.

The twenty-first to the twenty-sixth aspects relate to uses of BACE-1 comprised in a population of BDEVs. In a particular embodiment of any of the twenty-first to the twenty-sixth aspects, use of BACE-1 comprises determining a level of brain-derived BACE-1 present in an isolated sample obtained from a subject, particularly by determining the level of BACE-1 comprised in a population of BDEVs, more particularly wherein the population of BDEVs is captured on an insoluble support. As detailed above in particular embodiments of the first to the thirteenth aspects, determining the level of BACE-1 comprised in the population of BDEVs may be carried out by using an affinity reagent capable of specifically binding BACE-1. As also detailed above, capturing of the population of BDEVs may be carried out by using an affinity reagent capable of specifically binding a marker for the population of BDEVs, particularly an affinity reagent capable of specifically binding neuroligin-3 (NLGN3), more particularly wherein the affinity reagent is an antibody or a functional antibody fragment.

The twenty-sixth aspect refers to an *in vitro* method of determining a subject's probability of developing Alzheimer's disease within the 3-5 subsequent years. The method of the twenty-sixth aspect may comprise determining the presence and/or level of BACE-1 comprised in a population of brain-derived extracellular vesicles (BDEVs) in an isolated sample obtained from the subject. In this sense, the presence of BACE-1 or the level of BACE-1 determined in the population of BDEVs in the isolated sample obtained from the subject is indicative of the subject's probability of developing Alzheimer's disease within the 3-5 subsequent years. In particular, when BACE-1 is determined to be present in the population of BDEVs in the isolated sample obtained from the subject, or, alternatively, when the level of BACE-1 comprised in the population of BDEVs in the isolated sample obtained from the subject is higher than the level of BACE-1 comprised in a population of BDEVs in an isolated sample obtained from an age-matched control subject, this is indicative of a probability of developing Alzheimer's disease within the 3-5 subsequent years that is higher than the average probability determined for subjects of the same age group that the subject belongs to.

In particular embodiments of the present disclosure, determining the level of BACE-1 is carried out on an *in vitro* diagnostic device. Suitable *in vitro* diagnostic devices for use in the context of the present disclosure are known in the art, for example, as disclosed in WO2023006817A1. The entire contents of WO2023006817A1 are herein incorporated by reference. Thus, in more particular embodiments, determining the level of BACE-1 is carried out on an *in vitro* diagnostic device, wherein the *in vitro* diagnostic device comprises:
- an electrode (92, 102);
- a first cover (41) comprising
- a chamber (11, 411, 91) comprising
- a first opening (111, 911) for adding magnetic particles and liquids (45) into the chamber (11, 411, 91),
- and a second opening (112) for letting the magnetic particles and liquids contact the electrode (92, 102) placed in the device adjacent to the chamber (11, 411, 91);
- and a sealing element (12, 412);
- a second cover (42) fitting the first cover (41), the second cover (42) comprising
- a liquid absorbing pad (13, 421) adjacent to the chamber (11, 411, 91);
- a liquid exit (15, 43) for letting the liquid exit the chamber (11, 411, 91);
- an opening assembly (14, 46) for shifting the device between a closed position and an open position, the opening assembly (14, 46) comprising
- a first cooperating mechanism (461) and a second cooperating mechanism (462) wherein the first cover (41) comprises the first cooperating mechanism (461) and the second cover (42) comprises the second cooperating mechanism (462); wherein:
- the sealing element (12, 412) is for sealing the liquid exit (15, 43);
- the liquid absorbing pad (13, 421) faces at least part of the liquid exit (15, 43) of the chamber (11, 411, 91) in
the open position of the device; and wherein:
- in the closed position the sealing element (12, 412) covers the liquid exit (15, 43) such that a liquid is prevented from flowing out of the chamber (11, 411, 91) through the liquid exit (15, 43);
- and in the open position:
- at least part of the liquid exit (15, 43) is uncovered by the sealing element (12, 412) and - the device comprises a fluidic path (16, 44) between the chamber (11, 411, 91) and the liquid absorbing pad (13, 421) through the uncovered part of the liquid exit (15, 43). In any embodiment of any one of the first to the twenty-sixth aspects, the term "brain-derived extracellular vesicles" may alternatively be replaced with the terminology "extracellular vesicles derived from a brain cell type". In a more particular embodiment of the present disclosure, the brain cell type is a neuron cell or a glial cell, particularly a neuron cell. In another more particular embodiment, the brain cell type is not an astrocyte.

The *in vitro* methods of the disclosure generally provide for determining the diagnosis or classification of AD, MCI, and AD-associated dementia, memory decline and pathological changes in a subject, and, optionally, for recommending an appropriate medical regimen for the treatment of the patient in case of a positive diagnosis. In some embodiments, said method may further comprise the steps of (i) collecting the diagnostic information, and (ii) saving the information on a data carrier. In the sense of the invention a "data carrier" is to be understood as any means that contain meaningful information data for determining the diagnosis, in particular, for the early diagnosis of AD, in a subject and/or for recommending an appropriate medical regimen, such as paper. The carrier may also be any entity or device capable of carrying the data or information for recommending an appropriate therapy. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the diagnosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

In the present disclosure, all particular embodiments disclosed in relation to one aspect also represent, insofar as applicable, particular embodiments of all other aspects. In other words, a particular embodiment disclosed in relation to one aspect also represents, insofar as applicable, a particular embodiment of any one of the other aspects of the present disclosure.

### Examples

### Instrumentation and Chemical Reagents

Cell culture medium, Dulbecco's Modified Eagle Medium F12 (DMEM F12), with GlutaMAX supplement (ref. 31331028), and fetal bovine serum (FBS, ref. 26140079), were obtained from Gibco (Thermo Fisher Scientific). The chemicals for the preparation of all buffers and solutions, including dibasic sodium phosphate (ref. 71636), dibasic potassium phosphate (ref. 795496), sodium chloride (ref. S3014), potassium chloride (ref. P3911), boric acid (ref. B6768), glycine (ref. 50046), hydroquinone (ref. H9003), hydrogen peroxide (ref. 1072090500), and bovine serum albumin (BSA, ref. A4503), were purchased from Sigma-Aldrich (Merck KGaA, Darmstadt, DE). Sodium carbonate (ref. 131648) was obtained from Panreac (ES). All solutions were prepared using MilliQ ultrapure water (Millipore^{®} System, resistivity 18.2 MΩ·cm).

Flow cytometry was carried out employing a Cytoflex LX equipment (Beckman Coulter Inc, Indianapolis, IN, US). Media Fluorescence Intensity (MFI) and bead count data were extracted using FlowJo analysis software from each sample-reading file.

Optical and chemiluminescent measurements were executed using a TECAN Infinite^{®} M Plex, multimode microplate reader (Tecan Group Ltd, Männeford, CH) using i-Control^{™} software. Polypropylene microtiter plates for optical detection (magneto-ELISA) were purchased from Costar (Corning Inc., NY, US). OptiPlate-96 white microplates for chemiluminescent detection (magneto-CHEM-ELISA) were purchased from Perkin Elmer (Walthman, MA, US). The electrochemical readout was achieved on carbon screen-printed electrodes (ref. DRPC110) using a portable bipotentiostat DRP-STAT200 operated by DropView 200 for instrument control and data acquisition (Dropsens, Metrohm AG, CH). All data were subjected to nonlinear regression analysis (Four Parameter logistic Equation-GraphPad Prism Software) for fitting.

Tosylactivated magnetic particles (MPs) (Dynabeads M450 Tosylactivated, ref. 14013), the Pierce BCA Protein Assay Kit (ref. 23225), the Pierce TMB Substrate Kit (ref. 34021), the SuperSignal^{™} ELISA Pico Chemiluminescent Substrate (ref. 37070), the Poly-HRP Streptavidin (Strep-polyHRP) (ref. 21140), the antibodies: mouse monoclonal anti-CD9 (ref. 10626D), mouse monoclonal anti-CD81 (ref. 10630D), mouse monoclonal anti-CD63 (ref 10628D), mouse monoclonal anti-NLGN3 (ref. MA527621), mouse monoclonal anti-L1CAM (ref. 14-1719-82), rabbit polyclonal anti-GAP43 (ref. BS-0154R), rabbit polyclonal anti-Rab27b (ref. PA5-80962) rabbit polyclonal anti-BACE-1 (ref. A11034), rabbit monoclonal anti-CD81 (ref. MA532333), rabbit monoclonal anti-NLGN3 (ref. MA536008), and the secondary antibody goat anti-Rabbit IgG (H+L) Highly Cross-Adsorbed Alexa Fluor^{™} 488 (ref. A11034) were purchased from Thermo Fisher Scientific (Walthman, MA, US). Biotinylated antibody anti-BACE-1 (ref. ABIN7145532) was obtained from Antibodies-online Inc. (Pottstown, PA, US). Antibody mouse monoclonal anti-CD63-HRP (ref. NBP2-42225H) was purchased from Novus Biologicals (Bio-techne, Minneapolis, MN, US). Secondary antibody goat anti-mouse IgG (Cy5^{®}) (antimouse-Cy5) (Ref. ab97037) was obtained from Abcam (Cambridge, GB).

### Cell Culturing, Exosome Isolation and Purification

Differential ultracentrifugation was used to purify exosomes from the supernatant of SH-SY5Y cell line using methods as disclosed in Théry et al., "Isolation and Characterization of Exosomes from Cell Culture Supernatants and Biological Fluids", Current Protocols in Cell Biology, 2006, vol. 30, issue 1, pp. 3.22.1-3.22.29, with minor modifications as shown in FIG. 1. Briefly, cell culture supernatant was first centrifuged at 300 x g for 5 min if the procedure was done in fresh media (for stored supernatant this step was performed before freezing), allowing the removal of suspended cells, which were re-cultured, as SH-SY5Y cells are a mix of adherent and suspended cells, as disclosed by the American Type Culture Collection (ATCC) in the product data sheet for this cell line (ATCC CRL-2266; https://www.atcc.org/products/crl-2266). Supernatant was then centrifuged at 2,000 x g for 15 min, and at 10,000 x g for 30 min, allowing the removal of microvesicles and cell debris. A further ultracentrifugation at 100,000 x g for 60 min was performed. Finally, the supernatant was gently discarded and the pellet containing exosomes were resuspended in sterile-filtered (0.22 µm) PBS 1X, pooled, and the ultracentrifugation was repeated at 100,000 x g. The resulting exosome pellet was resuspended in sterile-filtered (0.22 µm) PBS 1X (500 µL per 200 mL of starting medium supernatant) and stored at -20°C. All centrifugation and ultracentrifugation steps were carried out at a temperature of 4°C.

### Characterisation of Isolated Exosomes by Nanoparticle Tracking Analysis (NTA) and Cryo-TEM

The isolated exosomes were analysed by nanoparticle tracking analysis (NTA) utilising the NanoSight LM10-HS system equipped with a calibrated 405 nm laser (NanoSight Ltd, Malvern, GB). NTA characterization was performed by recording raw data videos in triplicate for 60 seconds with 25 frames per second. The laser unit temperature was set to 24.8°C.

As shown in FIG. 2, the NTA analysis of exosomes derived from the SH-SY5Y cell line revealed a concentration of 1.59 x 10¹¹ (SD = 2.02 x 10⁹; SD = standard deviation) particles/mL, while the size distribution histogram (FIG. 2A) showed a main peak at 155 nm and a second smoother peak at 225 nm compatible with exosome sizes. Results of NTA analysis showed a similar size distribution across the three different pooled human plasma samples, with a main peak at 135 nm for both extracellular vesicles (EVs) from healthy donors (FIG. 2B) and MCI patients (FIG. 2C), and a main peak at 125 nm followed by a second peak at 185 nm for EVs from AD patients (FIG. 2D). The shape of the NTA trace of EVs from plasma of AD patients is the most similar one to the shape of the NTA trace of EVs from SH-SY5Y cells. All main peaks are compatible with exosome sizes. Average concentrations were: 3.48 x 10¹⁰ (SD 1.44 x 10⁹) particles/mL for EVs extracted from plasma of healthy donors, 7.64 x 10¹⁰ (SD 1.36 x 10⁹) particles/mL for EVs extracted from plasma of MCI patients and 1.63 x 10¹¹ (SD 6.43 x 10⁹) particles/mL for EVs extracted from plasma of AD patients.

To further characterise exosomes, cryogenic transmission electron microscopy (Cryo-TEM) images of isolated exosomes were acquired using a Jeol JEM 2011 transmission electron microscope (JEOL USA Inc, MA, US) operating at an acceleration voltage of 200 kV. Images obtained with Cryo-TEM showed particle sizes compatible with exosomes, and confirmed vesicle integrity in all the cases, as shown in the insets in FIG. 2.

### Characterization of Total Protein Content by Bicinchoninic Acid Assay (BCA) Protein Assay Kit

Total protein content of purified exosomes was performed using a BCA protein assay kit according to the instructions provided by the manufacturer. Briefly, Bovine Serum Albumin (BSA) standard solutions were prepared, by diluting BSA in PBS 1X, in a working range from 0 to 2000 µg/mL. Then, 10 µL of each standard or exosome sample was pipetted (3 replicates) into a microplate well and 190 µL of the BCA reagent (Reagent A: Reagent B, 50:1, v/v) were added. The plate was then incubated at 37°C for 30 minutes and the absorbance was read at 562 nm on the microplate reader. From BCA assay, the total protein content of SH-SY5Y derived exosomes was estimated at 626 µg/mL (SD = 86 µg/mL).

### Flow Cytometry Analysis of SH-SY5Y Cell Surface Markers

Analysis of the expression of CD81 (Uniport ID: P60033), BACE-1 (Uniprot ID: P56817), NLGN3 (Uniprot ID: Q9NZ94), L1CAM (Uniprot ID: P32004), and GAP-43 (Uniprot ID: P17677) on the surface of SH-SY5Y cells was performed by flow cytometry. The indirect labelling of 3 x 10⁵ cells was performed by incubation of 100 µL (5 µg/mL) of the antibodies anti-CD81 (mouse), anti-L1CAM (mouse), anti-NLGN3 (rabbit), and anti-GAP-43 (rabbit). A 30-minute incubation at 4°C was performed. After that, three washing steps with PBS pH 7.5 0.5% FBS were performed. Afterwards, cells were incubated with 100 µL (2 µg/mL) of anti-mouse-Cy5 antibody or 100 µL (2 µg/mL) of anti-rabbit-PE antibody for 30 min at 25°C. The labelled cells were resuspended in PBS and analysed by flow cytometry. Results are presented in FIG. 3, as both dot plots and histograms.

As shown in FIG. 3, a positive labelling was obtained for CD81 (99% positively labelled cells), which was used as positive control, as well as for L1CAM (92% positively labelled cells) and the markers GAP-43 (51% positively labelled cells), NLGN3 (44% positively labelled cells) and BACE-1 (43% positively labelled cells), thus confirming the presence of these markers on the surface of SH-SY5Y cells.

### Flow Cytometry Analysis of SH-SY5Y-Derived Exosomes by Direct Covalent Immobilisation of Exosomes on Magnetic Particles (Direct Immobilisation Assay)

In order to enable their analysis by flow cytometry, exosomes derived from SH-SY5Y cells were immobilised on magnetic particles through direct covalent immobilisation, using methods known in the art, as disclosed in Moura et al., "Multiplex detection and characterization of breast cancer exosomes by magneto-actuated immunoassay", Talanta, 2020, vol. 211, pp. 120657, and in Pallares-Rusiñol et al., "Chapter Two - Advances in Exosome Analysis", Advances in Clinical Chemistry, 2023, vol. 112, pp. 69-117. Briefly, exosomes were immobilised on tosylactivated superparamagnetic Dynabeads^{®} M450 particles (MPs) by adding 3.5 x 10¹⁰ exosomes to 40 µL of MPs (containing 1.6 x 10⁷ MPs). A 0.1 M borate buffer, pH 8.5, was added to ensure the nucleophilic reaction by the amine group of exosomes membrane proteins and to improve the reaction kinetics. The incubation step was carried out overnight at 4°C with gentle agitation. Then, a 0.5 M glycine solution (blocking agent) was added and incubated for 2 hours at 25°C to block (deactivate) any remaining free tosyl groups. The exosome-modified MPs were then resuspended in 160 µL of 10 mM PBS buffer to obtain 1 x 10⁶ MPs per 10 µL.

Magnetic particle-based flow cytometry was used to characterise possible targets present on the membrane of SH-SY5Y-derived exosomes. Specifically, the following were evaluated: (i) the presence of the neuronal marker proteins NLGN3 and L1CAM, (ii) the presence of AD marker proteins BACE-1, GAP-43 and Rab27b, and (iii) the presence of classical exosomal markers, namely tetraspanins CD9, CD63 and CD81. After immobilisation of exosomes on MPs, rabbit primary antibodies anti-NLGN3 (1.0 µg/ well), anti-GAP-43 (1.0 µg/well), anti-BACE-1 (3.0 µg/well), anti-Rab27b (2.0 µg/ well), anti-CD81 (2.0 µg/well), and mouse primary antibodies anti-L1CAM (2.0 µg/well), anti-CD9 (2.0 µg/well) and anti-CD63 (2.0 µg/well) were added and incubated for 1 hour with gentle shaking at 25°C, followed by washing three times with PBS 1X containing 0.5% BSA (150 µL/well). For detection, 100 µL of goat anti-rabbit secondary antibody conjugated with Alexa Fluor^{™} 488 (2.0 µg/mL) or 100 µL of goat anti-mouse secondary antibody conjugated with Cy5 (2.0 µg/mL), as applicable depending on the species of the primary antibody, were added and incubated for 30 min in the dark with gentle shaking at 25°C. Incubation was followed by three washing steps with PBS 1X containing 0.5% BSA (150 µL/well), after which exosome-modified MPs (exosome-MPs) were resuspended in 200 µL PBS 1X for the injection into the flow cytometer. Magnetic separation was performed after each incubation or washing step by placing a magnet under the microtiter plate until exosome-MP pellets formed in the lower corner, followed by removal of the supernatant.

A schematic diagram representing an exosome-modified MP is shown in FIG. 4, upper left corner. Also shown on this diagram is a primary antibody binding to a target present on the membrane of an exosome immobilised on said MP, which may be an exosome surface marker or may be an exosome cargo protein. The skilled person is aware that, when such targets present on the membrane of an exosome are detected in exosomes that have not been exposed to detergents or other conditions that may disrupt or damage exosome membranes, the antibody or other affinity reagent that binds such targets will bind to a region of the target that is exposed externally on the surface of exosomes.

Results of the flow cytometry evaluation of the presence of different surface markers on the membrane of SH-SY5Y-derived exosomes directly covalently immobilised on MPs are presented in FIG. 4 as dot plots. As shown in FIG. 4, the membrane of SH-SY5Y-derived exosomes is enriched in exosome-marker tetraspanins, particularly CD9 and CD63, with a positive labelling of 96.1% and 95.7% respectively, followed by CD81 at 36.8%. Positive labelling was also observed for the neuronal markers NLGN3 (98.5%) and L1CAM (59.1%), as well as for the AD markers BACE-1 (65.4%) and GAP-43 (81.9%). A lower percentage of positive labelling was detected for Rab27b (6.47%).

### Flow Cytometry Analysis of SH-SY5Y-derived Exosomes Captured with Antibodies Covalently Bound to Magnetic Particles (Antibody Capture Assay)

To immobilise capture antibodies on the surface of tosylactivated magnetic particles, 11 µg/mL of each capture antibody was added to 40 µL (=1.6 x 10⁷ MPs) of tosylactivated Dynabeads^{®} M450 particles. To increase reaction kinetics, 150 µL of 0.1 M borate buffer pH 8.5 and 100 µL of 3M ammonium sulphate were added. The incubation step was carried out overnight at 37°C with gentle agitation. Next, the MPs were incubated for 4 hours with the blocking agent, 0.5 M glycine solution, at 37°C, to block any remaining free tosyl groups. Subsequently, the antibody-modified MPs were resuspended in 160 µL of 10 mM PBS buffer to obtain 1 x 10⁶ MPs per 10 µL.

Polypropylene microtiter plates (96 wells) were used for the capture of SH-SY5Y-derived exosomes on antibody-modified MPs (giving rise to MP-antibody-exosome complexes) and their subsequent characterisation by magnetic particle-based flow cytometry. Briefly, exosomes were captured using MPs modified with different capture antibodies, according to the protocol described above (herein referred to as antiX-MPs, wherein X is a protein marker selected from: CD9, CD81, L1CAM, BACE-1 and NLGN3), by incubating 1.0 x 10⁶ antiX-MPs/well and 100 µL (=4.4 x 10⁷ exosomes/µL) of SH-SY5Y-derived exosomes, for 1 hour with gentle agitation at 25°C. After incubation, the solution was discarded, and MP-antibody-exosome complexes were washed with PBS 1X solution containing 0.5% BSA (150 µL/well). Subsequently, the presence and levels of different membrane proteins on SH-SY5Y-derived exosomes were evaluated by labelling MP-antibody-exosome complexes with different primary antibodies. For this, a primary antibody anti-Y (wherein Y is a protein marker selected from: CD9, CD81, L1CAM, BACE-1, NLGN3, GAP-43 and Rab27b; anti-Y antibodies were used at concentrations ranging from 0.5 to 3 µg/well) was diluted in PBS 1X containing 0.1% BSA, and incubated with MP-antibody-exosome complexes for 45 minutes at 25°C in microtiter plate wells. Following the incubation period, wells were washed three times with PBS 1X containing 0.5% BSA. Subsequently, 100 µL of goat anti-rabbit secondary antibody conjugated with Alexa Fluor^{™} 488 (2.0 µg/mL) or 100 µL of streptavidin conjugated with Alexa Fluor^{™} 488 (2.0 µg/mL) were added and incubated for 30 min in the dark with gentle shaking at 25°C. Incubation with the secondary antibody or streptavidin was followed by three washing steps with PBS 1X containing 0.5% BSA (150 µL/well) and the labelled MP-antibody-exosome complexes were then resuspended in 200 µL PBS 1X for injection into the flow-cytometer. Magnetic separation was performed after each incubation or washing step by placing a magnet under the microtiter plate until the MP-antibody-exosome pellets formed in the lower corner, to allow removal of the supernatant.

The results of these experiments, as well as the results of the characterisation of SH-SY5Y cells and of directly covalently immobilised exosomes by flow cytometry, are summarised in FIG. 5. The data in FIG. 5 demonstrate that the combination of NLGN3, as a marker used to specifically capture brain-derived EVs, in particular brain-derived exosomes, and BACE-1, as an AD marker present on the surface of the captured exosomes, may be particularly advantageous for use in diagnostic methods, particularly in diagnostic methods comprising magneto-immunoassays. In particular, the use of anti-NLGN3 antibody as a capture antibody coupled to MPs in combination with detection of BACE-1 on the captured exosomes using biotinylated anti-BACE-1 followed by streptavidin-AlexaFluor488 advantageously resulted in a low background and a positive labelling of the MP-antiNLGN3-exosome complexes of 14.7%. Advantageously, streptavidin-polyHRP may be used to amplify the signal and further improve sensitivity.

Surprisingly, the inventors found that expression of NLGN3 was higher on membrane of SH-SY5Y-derived exosomes than on the membrane of parental cells, demonstrating that NLGN3 is enriched on the membrane of exosomes. The opposite was observed for L1CAM.

### Evaluating the capture capabilities of antiNLGN3-MPs

Further experiments were performed with magnetic particles modified with antibodies against the tetraspanin CD9 - a pan-exosome marker used as a positive control - and NLGN3 in order to assess the capability of anti-NLGN3 antibodies coupled to MPs to capture, separate and preconcentrate exosomes. Detection of the captured exosomes was performed by detecting the tetraspanin CD63, a classical exosome marker, using an HRP-coupled anti-CD63 antibody (antiCD63-HRP) through carrying out magneto-ELISAs with optical (FIG. 6A; absorbance at 450 nm) and chemiluminescent (FIG. 6B) readouts. Calibration plots were fitted using a non-linear regression (four-parameter logistic equation) (n=3).

As shown in FIG. 6, exosomes were successfully captured with both antiCD9-MPs and with antiNLGN3-MPs, and the intensity of the signal (plotted on the y-axis) arising from the detection of CD63 on the surface of the captured exosomes was positively correlated with the concentration of exosomes (plotted on the x-axis) in the original exosome sample. The same pattern of detection was observed for both optical (FIG. 6A) and chemiluminescent (FIG. 6B) readouts.

When antiNLGN3-MPs were used, the regression coefficients (R²) of the fittings obtained by four parameters logistic (4PL) equation were 0.9758 for the optical readout and 0.9979 for the chemiluminescent readout, indicating high fitting and reliability of the assays, with a limit of detection (LOD) of 7.13 x 10⁶ exosomes and 7.83 x 10⁶ exosomes for magneto-ELISA and magneto-CHEM-ELISA respectively. When antiCD9-MPs were used, the R2 obtained by the 4PL equation were 0.9805 for the optical read out and 0.9893 for the chemiluminescent readout, indicating high fittings and reliability of the assays, similarly to what observed with antiNLGN3-MPs. The LOD for antiCD9-MPs were 4.91 x 10⁶ exosomes for the ELISA and 6.92 x 10⁵ exosomes for the magneto-ELISA. As CD9 is a generic exosome marker, a higher capture was obtained with antiCD9-MPs. Nevertheless, both modified MPs showed similar performances overall, across the two readout systems. Specifically, antiNLGN3-MPs showed good capture capabilities, with the advantage of capturing selectively brain-derived exosomes (BDEs).

### Magneto-actuated immunoassays for the quantification of BACE-1 on brain-derived exosomes (BDEs)

Magneto-actuated immunoassays with optical, chemiluminescent and electrochemical readouts were carried out to detect and evaluate BACE-1 present in exosomes derived from SH-SY5Y cells captured using antiNLGN3-MPs. FIG. 7A summarises the principles underlying the magnetic capture methodology used in magneto-actuated immunoassays. In these assays, tosylated magnetic particles functionalised with an anti-NLGN3 antibody (antiNLGN3-MPs) were used to capture brain-derived exosomes (BDEs). Advantageously, detection and quantification of BACE-1 on captured BDEs used a biotinylated anti-BACE-1 antibody in combination with streptavidin-poly-HRP (Strep-Poly-HRP) to enhance the signal. The signal was then detected on optical, chemiluminescent and electrochemical detection platforms.

The data corresponding to the normalised calibration plots for optical (i.e. absorbance at 450 nm), chemiluminescent and electrochemical readouts are shown in FIG. 7B. For this, the calibration plots were constructed for different exosome concentrations, ranging from 2.03 x 10⁴ to 4.4 x 10⁷ exosomes/µL for the optical and the chemiluminescent readout and from 2.03 x 10⁴ to 1.45 x 10⁷ exosomes/µL for the electrochemical readout. In all cases, the values were fitted using a non-linear regression (four-parameter logistic equation). The limits of detection (LOD) were 1.91 x 10^{4.5} exosomes µL⁻¹ (R²=0.9791), 1.64 x 10⁵ exosomes/µL (R²=0.9598), and 1.51 x 10⁴ exosomes µL⁻¹ (R²=0.9829), respectively, for the magneto-actuated immunoassays with optical, chemiluminescent and electrochemical readout, as indicated on the graph in FIG. 7B. As shown in FIG. 7B, the three platforms gave overall comparable results, with a high fitting indicating that the assays were highly reliable and robust. Of these, the electrochemical readout method resulted in a lower LOD and thus may represent the more sensitive readout platform.

The electrochemical readout, also known as amperometric readout, was performed using a prototype device apt for point-of-care (PoC) use. Specifically, the electrochemical readout was based on amperometry using carbon screen-printed electrodes (ref. DRP-C110, DropSens, Metrohm AG CH), with an electrochemical cell consisting of a working and auxiliary electrode made of carbon, and a reference electrode made of silver. A permanent neodymium magnet was placed above the electrode. The small dimensions (L 33 x W 10 x H 0.5 mm) of the electrodes are adapted to work both with microvolumes of samples, as well as in solution.

In the case of the electrochemical readout platform, electrodes were connected to the boxed connector for SPE (Ref. DSC), operating as an interface between the electrodes and the portable bipotentiostat (DRP-STAT200, DropSens, Metrohm AG). An amperometric readout was performed using a potential of -0.12 V (vs Ag pseudo-reference) in ePBS buffer (0.1 M Na₂HPO₄, 0.1 M KCI, pH 7.0) (150 µL) in presence of 2 mM hydrogen peroxide and 2 mM hydroquinone, after 10 minutes incubation under gentle agitation. The electrodes were discarded after each measurement. A reproducible steady-state current was obtained after 30 s (as can be seen in FIG. 8A, as well as in FIG. 9B). The benzoquinone species was monitored, and the cathodic current generated was directly related to the number of captured exosomes. The Biotin-antiBACE-1 labelled with the HRP conjugate, was used as electrochemical reporter in the presence of hydrogen peroxide (H₂O₂) as a substrate of HRP and hydroquinone (HQ) as a mediator. HRP catalyses the transfer of two electrons from HQ to H₂O₂ to generate water and benzoquinone (BQ), the oxidized form of HQ. First, the HRP is oxidized catalysing the reduction of H₂O₂ to H₂O, then the HRP is reduced again by oxidizing HQ to BQ (FIG. 8B).

Thus, for the electrochemical readout, the final readout at the surface of the electrode is based on the reduction of the BQ to HQ. Since the enzyme HRP works at saturated substrate conditions, it also works at maximum speed and turn-over range, providing a directly proportional signal to the sample (hence the higher the number of exosomes/Biotin-antiBACE-1/HRP conjugate, the higher the signal). The amperometric measurements were performed on a laptop computer in which the portable bipotentiostat was connected by a universal USB port.

FIG. 8A shows the raw chronoamperograms (average of 3 replicates) for exosomes derived from SH-SY5Y cells ranging from 2.03 x 10⁴ to 1.45 x 10⁷ exosomes/µL. These data demonstrate that the amperometric readout, i.e. intensity of the detected current (µA), is directly correlated with the concentration of BACE-1-carrying exosomes in the sample.

### Collection and Processing of Human Plasma Samples

Plasma samples from patients suffering from Mild Cognitive Impairment (MCI), N=5, and Alzheimer's disease (AD), N=5, obtained from ACE Alzheimer Center Barcelona, were pooled. Plasma samples from healthy donors, N=50, obtained from *Banc de Sang i Teixits,* Barcelona, were pooled (to diminish differences between control sera) and used as control (CTRL). Exosome extraction and purification was obtained by differential ultracentrifugation, similarly to the method for exosome isolation outlined above and in FIG. 1, with minor modifications, as described in Cano et al., "Plasma extracellular vesicles reveal early molecular differences in amyloid positive patients with early-onset mild cognitive impairment", Journal of Nanobiotechnology, 2023, vol. 21, issue 1, pp. 54. Briefly, the procedure involved an initial centrifugation for 30 min at 10,000 x g, followed by two sequential ultracentrifugation steps performed at 100,000 x g for 1 hour each. The resulting exosome pellet was resuspended in sterile-filtered (0.22 µm) PBS 1X (50 µL PBS 1X per 3 mL of starting plasma).

Plasma exosomes from CTRL subjects, MCI patients and AD patients were analysed using a magneto-actuated assay, to capture brain-derived exosomes (BDEs) using an anti-NLGN3 antibody, coupled with an electrochemical readout platform, to detect and quantify BACE-1 present on the captured BDEs in each sample. Detect and quantification of BACE-1 was performed using a biotinylated anti-BACE-1 antibody in combination with streptavidin-poly-HRP (Strep-Poly-HRP) to enhance the signal, as described above for SH-SY5Y-derived exosomes. In particular, the analysis of exosomes from human plasma was carried out using a miniaturised portable device operated by batteries integrated with a screen-printed electrode apt for Point-of-Care (PoC) use.

As shown in FIG. 9, higher signal was obtained for AD patients compared to MCI, which, in turn showed higher signal than control. These results demonstrate that, through implementing a method according to particular embodiments of the present disclosure, which comprises the quantification of BACE-1 in brain-derived exosomes (BDEs) in human plasma samples, patients with dementia (MCI and AD) could be clearly discriminated from CTRL subjects. Furthermore, within the group of patients with dementia, the assay could discriminate between patients with MCI and patients with AD. These results demonstrate the assay's value in diagnosing AD, as well as MCI, as well as stratifying patients and identifying patients who are at risk of developing AD.

### Citation List

### Patent literature:

### WO2023006817A1

### Non-Patent Literature:

Braak et al., "Staging of Alzheimer disease-associated neurofibrillary pathology using paraffin sections and immunocytochemistry", Acta Neuropathologica, 2006, vol. 112, issue 4, pp. 389-404
Cano et al., "Plasma extracellular vesicles reveal early molecular differences in amyloid positive patients with early-onset mild cognitive impairment", Journal of Nanobiotechnology, 2023, vol. 21, issue 1, pp. 54
Clark et al., "Polygenic Risk Scores in Alzheimer's Disease Genetics: Methodology, Applications, Inclusion, and Diversity", Journal of Alzheimer's Disease, 2022, vol. 89, issue 1, pp. 1-12
Comfort et al., "Nanoparticle Tracking Analysis for the Quantification and Size Determination of Extracellular Vesicles", 2021, Journal of Visualized Experiments, vol. 169, pp: e62447
Cummins et al., "Alzheimer's disease drug development pipeline: 2024", Alzheimer's & Dementia, 2024, vol. 10, pp: e12465
Huang et al., "Clinical trials of new drugs for Alzheimer disease: a 2020-2023 update", Journal of Biomedical Sciences, 2023, vol. 30, issue 1, pp: 83
Jack et al., "NIA-AA Research Framework: Toward a biological definition of Alzheimer's disease", Alzheimer's & Dementia, 2018, vol. 14, issue 4, pp. 535-562
Jack et al., "Revised criteria for diagnosis and staging of Alzheimer's disease: Alzheimer's Association Workgroup", Alzheimer's & Dementia, 2024, vol. 20, issue 8, pp. 5143-5169
Khan et al., "Determining the Size Distribution and Integrity of Extracellular Vesicles by Dynamic Light Scattering", 2022, Methods in Molecular Biology, vol. 2413, pp. 165-175
Leonenko et al., "Identifying individuals with high risk of Alzheimer's disease using polygenic risk scores", Nature Communications, 2021, vol. 12, article number 4506
Miyakawa-Liu et al., "Rates of Cognitive Decline in 100 Patients With Alzheimer Disease", Ochsner J, 2022, vol. 22, issue 2, pp. 129-133
Moura et al., "Multiplex detection and characterization of breast cancer exosomes by magneto-actuated immunoassay", Talanta, 2020, vol. 211, pp. 120657
Pallares-Rusinol et al., "Chapter Two - Advances in exosome analysis", Advances in Clinical Chemistry, 2023, vol. 112, pp. 69-117
Pemberton et al., "Quantification of amyloid PET for future clinical use: a state-of-the-art review", Eur J Nucl Med Mol Imaging, 2022, vol. 49, issue 10, pp. 3508-3528
Théry et al., "Isolation and Characterization of Exosomes from Cell Culture Supernatants and Biological Fluids", Current Protocols in Cell Biology, 2006, vol. 30, issue 1, pp. 3.22.1-3.22.29
Zhang et al., "Comprehensive isolation of extracellular vesicles and nanoparticles", Nature Protocols, 2023, vol. 18, pp. 1462-1487

## Claims

1. An *in vitro* method for: (i) deciding or recommending whether a subject should undergo testing for amyloid, Tau and neuronal degeneration biomarkers required for classification of the subject according to the A/T/N system, or, alternatively, (ii) classification of a subject as A+ (amyloid positive) or A- (amyloid negative) according to the A/T/N system, or, alternatively, (iii) predicting a pathological rate of memory decline in a subject without dementia, or, alternatively, (iv) identifying a subject with Alzheimer's disease-associated pathological changes,
the method comprising a step of determining the level of β-secretase 1 protein (BACE-1) comprised in a population of brain-derived extracellular vesicles, wherein the population of brain-derived extracellular vesicles carry neuroligin-3 (NLGN3), in an isolated sample obtained from the subject,
wherein when the level of BACE-1 is equal to or higher than a reference this is indicative of, respectively: (i) a positive decision or positive recommendation that the subject undergoes testing for amyloid, Tau and neuronal degeneration biomarkers required for classification of the subject according to the A/T/N system, or, alternatively, (ii) a classification as A+ of the subject according to the A/T/N system, whereas when the level of BACE-1 is lower than the reference this is indicative of a classification as A- of the subject according to the A/T/N system, or, alternatively, (iii) a pathological rate of memory decline in the subject, or, alternatively, (iv) Alzheimer's disease-associated pathological changes in the subject.

2. An *in vitro* method for diagnosing Alzheimer's disease in a subject, the method comprising a step of determining the level of β-secretase 1 protein (BACE-1) comprised in a population of brain-derived extracellular vesicles, wherein the population of brain-derived extracellular vesicles carry neuroligin-3 (NLGN3), in an isolated sample obtained from the subject, wherein when the level of BACE-1 is equal to or higher than a reference this is indicative of a positive diagnosis of Alzheimer's disease.

3. The *in vitro* method according to claims 1 to 2, wherein the isolated sample obtained from the subject is a biological fluid selected from a list consisting of: blood, serum, plasma, lymph, cerebrospinal fluid, saliva, and urine, particularly wherein the isolated sample obtained from the subject is blood, serum, or plasma.

4. The *in vitro* method according to claims 1 to 3, wherein the step of determining the level of BACE-1 comprised in the population of brain-derived extracellular vesicles (BDEVs) is carried out using an anti-BACE-1 antibody or an anti-BACE-1 functional antibody fragment

5. The *in vitro* method according to claims 1 to 4, wherein the method comprises the following steps: (i) capturing, particularly onto an insoluble support, more particularly onto magnetic particles, of the population of brain-derived extracellular vesicles (BDEVs) comprised within the isolated sample; and (ii) determining the level of BACE-1 comprised in the population of brain-derived extracellular vesicles (BDEVs) captured in step (i).

6. The *in vitro* method according to claim 5, wherein step (i) is carried out using an affinity reagent that binds a marker for a population of brain-derived extracellular vesicle (BDEVs), wherein the marker is a membrane protein enriched in a brain cell type, particularly wherein the marker is a neuron-enriched membrane protein, particularly step (i) is carried out using an affinity reagent that binds neuroligin-3 (NLGN3), more particularly is step (i) carried out using an anti-NLGN3 antibody or anti-NLGN3 functional antibody fragment.

7. The *in vitro* method according to claims 1 to 6, wherein the population of brain-derived extracellular vesicles (BDEVs) comprises brain-derived exosomes, particularly wherein the number of brain-derived exosomes represents at least 80% of the total number of extracellular vesicles in the population of BDEVs.

8. A kit or device comprising: a) means for capturing a population of brain-derived extracellular vesicles; b) means for detecting the presence and/or for determining the level of BACE-1 protein; and optionally, c) a solid support.

9. The kit or device according to claim 8, wherein the means for capturing the population of brain-derived extracellular vesicles comprises an affinity reagent capable of specifically binding to a marker for brain-derived extracellular vesicles (BDEVs), particularly comprises an anti-NLGN3 antibody or anti-NLGN3 functional antibody fragment, more particularly comprises an anti-NLGN3 antibody or anti-NLGN3 functional antibody fragment immobilised on an insoluble support, even more particularly comprises an anti-NLGN3 antibody or anti-NLGN3 antibody fragment immobilised on magnetic beads.

10. The kit or device according to claims 8 to 9, wherein the means for detecting the presence and/or for determining the level of BACE-1 protein comprises an affinity reagent capable of specifically binding to BACE-1, particularly comprises an anti-BACE-1 antibody or anti-BACE-1 functional antibody fragment.

11. Use of a kit or device according to claims 8 to 10, in an *in vitro* method as defined in any one of claims 1 to 7.

12. Combined use of BACE-1 protein and of a marker for a population of brain-derived extracellular vesicles in an *in vitro* method of diagnosing Alzheimer's disease, wherein the marker for the population of brain-derived extracellular vesicles is a membrane protein enriched in a brain cell type, particularly wherein the marker for the population of brain-derived extracellular vesicles is a neuron-enriched membrane protein, more particularly wherein the marker for the population of brain-derived extracellular vesicles is Neuroligin-3 (NLGN3).

13. Combined use of BACE-1 and Neuroligin-3 (NLGN3) proteins in an *in vitro* method of diagnosing Alzheimer's disease.

14. Use of BACE-1 protein comprised in a population of brain-derived extracellular vesicles (BDEVs), as a biomarker for diagnosing Alzheimer's disease.

15. The use of BACE-1 according to claim 14, wherein the population of brain-derived extracellular vesicles (BDEVs) comprising BACE-1 carry neuroligin-3 (NLGN3) or a fragment thereof.
